(19) Europäisches Patentamt
European Patent Office
Office européen des brevets

(11) Publication number: **0 403 957 A2**

# (12) EUROPEAN PATENT APPLICATION

(21) Application number: 90111231.8

(22) Date of filing: 13.06.90

(51) Int. Cl.[5]: C07D 401/12, C07D 401/14, C07D 405/14, C07D 409/14, C07D 413/14, A61K 31/44

(30) Priority: 17.06.89 JP 153591/89

(43) Date of publication of application: 27.12.90 Bulletin 90/52

(84) Designated Contracting States: CH DE FR GB IT LI

(71) Applicant: FUJIREBIO INC.
6-7, Shimoochiai 4-chome Shinjuku-ku
Tokyo 161(JP)

(72) Inventor: Ikawa, Hiroshi
26-10, 1-chome Miyasaka, Setagaya-ku
Tokyo(JP)
Inventor: Kadoiri, Akiyoshi
1458-5-303, Hazama-cho
Hachioji-shi, Tokyo(JP)
Inventor: Sekine, Yasuo
23-7, 2-chome Fujizuka, Kohoku-ku
Yokohama-shi, Kanagawa-ken(JP)

(74) Representative: Dipl.-Ing. Schwabe, Dr. Dr. Sandmair, Dr. Marx
Stuntzstrasse 16
D-8000 München 80(DE)

## (54) Optical active 1,4-dihydropyridine derivates.

(57) Optical active 1,4-dihydropyridine derivatives of formula:

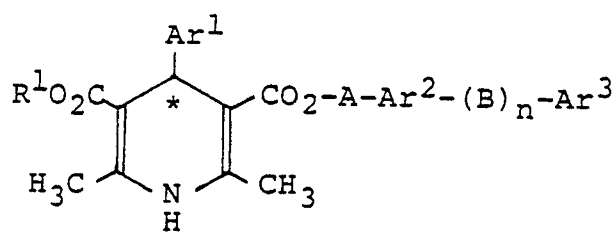

wherein $Ar^1$ represents an aromatic hydrocarbon group or an aromatic heteromonocyclic or heterobicyclic group containing therein 1 to 3 atoms selected from the group consisting of oxygen, sulfur and nitrogen; $R^1$ represents a hydrocarbon group which may have one or more substituents; A represents (i) a straight chain or branched chain unsaturated hydrocarbon group, (ii) a cyclic unsaturated hydrocarbon group, or (iii) a group selected from the group consisting of -R-O-N=CH-, -R-N=N-, -R-CH=N- and -R-N=CH-, in which R is an alkylene group having 1 to 6 carbon atoms; B represents an alkylene or alkenylene group having 1 to 3 carbon atoms, which may have a substituent; $Ar^2$ represents an aromatic hydrocarbon group or a heterocyclic group; $Ar^3$ represents a heterocyclic group which may have one or more substituents; and n is 0 or 1, and * indicates an optically active cite.

EP 0 403 957 A2

## OPTICAL ACTIVE 1,4-DIHYDROPYRIDINE DERIVATIVES

### BACKGROUND OF THE INVENTION

The present invention relates to optical active 1,4-dihydropyridine derivatives having superior vasodilative and platelet aggregation inhibiting activities.

Many derivatives of 1,4-dihydropyridine are known, which are remedies for diseases of circulatory system such as remedies for ischemic heart disease, cerebral circulatory disease and hypertension. For example, 1,4-dihydro-2,6-dimethyl-4-(2-nitrophenyl)pyridine-3,5-dicarboxylic acid dimethyl ester (U.S Patent No. 3,644,627; hereinafter referred to as "NIFEDIPINE") and 1,4-dihydro-2,6-dimethyl-4-(3-nitrophenyl)-pyridine-3,5-dicarboxylic acid-3-[2-(N-benzyl-N-methylamino)ethyl]ester-5-methyl ester hydrochloride (Japanese Patent Publication No. 45075/1980; hereinafter referred to as "NICARDIPINE") have already been utilized as hypotensor and coronary vasodilator. Moreover, other derivatives of 1,4-dihydropyridine having vasodilative activity together with inhibitory activity on platelet aggregation have been disclosed in Japanese Patent Applications as listed below:

Japanese Patent Application Laid-Open Nos. 140989/1981, 215684/1985, 226876/1985, 5076/1986, 10576/1986, 47477/1986, 197578/1986, 212581/1986 and 187468/1987.

1,4-dihydropyridine derivatives such as NIFEDIPINE and NICARDIPINE have high vasolidating activity, but they are unsatisfactory as remedy for arteriosclerosis. Therefore, researches are being made for obtaining 1,4-dihyropyridine derivatives having vasodilating activity together with platelet aggregation-inhibiting activity, capable of inhibiting platelet aggregation and curing arteriosclerosis. However, satisfactory remedies having the above-mentioned activities have not been found yet.

### SUMMARY OF THE INVENTION

It is therefore an object of the present invention to provide medicines having higher inhibitory activity on platelet aggregation together with vasodilative activity.

Another object of the present invention is to provide medicines for vasodilation and inhibition of platelet aggregation, which have a low toxicity and do not exhibit significant side effects.

A further object of the present invention is to provide medicines for vasodilation and inhibition of platelet aggregation, which are particularly effective for preventing and curing arteriosclerosis.

These objects of the present invention can be achieved by optical active 1,4-dihydropyridine derivatives having general formula (I):

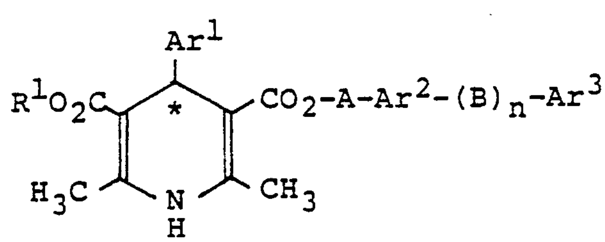

(I)

* optically active cite

wherein $Ar^1$ represents an aromatic hydrocarbon group or an aromatic heteromonocyclic or heterobicyclic group containing therein 1 to 3 atoms selected from the group consisting of oxygen, sulfur and nitrogen; $R^1$ represents a hydrocarbon group which may have one or more substituents; A represents (i) a straight chain or branched chain unsaturated hydrocarbon group, (ii) a cyclic unsaturated hydrocarbon group, or (iii) a group selected from the group consisting of -R-O-N=CH-, -R-N=N-, -R-CH=N-, and -R-N=CH-, in which R is an alkylene group having 1 to 6 carbon atoms; B represents an alkylene or alkenylene group having 1 to 3 carbon atoms, which may have a substituent; $Ar^2$ represents an aromatic hydrocarbon group or a heterocyclic group; $Ar^3$ represents a heterocyclic group which may have one or more substituents; and n is

0 or 1.

DETAILED DESCRIPTION OF THE PREFERRED EMBODIMENTS

In the optical active 1,4-dihydro-pyridine derivatives having the above general formula (I), $Ar^1$ may be an aromatic hydrocarbon group such as a phenyl group and a naphthyl group. Of these groups, a phenyl group is preferable as the aromatic hydrocarbon group represented by $Ar^1$.

Furthermore, $Ar^1$ may be an aromatic heteromonocyclic or heterobicyclic group containing therein 1 to 3 atoms selected from the group consisting of oxygen, sulfur and nigrogen (hereinafter referrerd to as the heterocyclic group), such as a pyridyl group, a benzoxazolyl group, a quinolyl group, a furyl group, a thienyl group, an isoquinolyl group, a benzthiazolyl, a benzoxadiazolyl and a benzthiadiazolyl. Of these groups, a pyridyl group, a benzoxazolyl group, a quinolyl group, a furyl group, and a thienyl group are preferable as the heterocyclic group represented by $Ar^1$.

The aromatic hydrocarbon groups or heterocyclic groups represented by $Ar^1$ may have one or more substituents selected from the group consisting of a nitro group, a trihalomethyl group such as trifluoromethyl and trichloromethyl, a cyano group, a halogen such as fluorine, chlorine, bromine and iodine, a phenoxy group, an azido group, a lower alkoxyl group having 1 to 4 carbon atoms, a lower alkyl group having 1 to 4 carbon atoms, a lower alkylthio group, a lower alkoxycarbonyl group, and a lower alkylcarbonyl group. Of these substituents, a nitro group, a trihalomethyl group such as trifluoromethyl and trichloromethyl, a cyano group, a halogen such as fluorine, chlorine, bromine and iodine, a phenoxy group are preferable as the substituents of the aromatic hydrocarbon groups or heterocyclic groups represented by $Ar^1$.

Specific preferable examples of the aromatic hydrocarbon group or heterocyclic group represented by $Ar^1$ are 2-nitrophenyl group, 3-nitrophenyl group, 4-nitrophenyl group, 2-cyanophenyl group, 2,3-dichlorophenyl group, 2-furyl group, 2-thienyl group, 3-thienyl group, 1-naphthyl group, 4-quinolyl group, 3-azidophenyl group, 3-trifluoromethylphenyl group, 4-methoxyphenyl group, 4-methylthiophenyl group, 3-pyridyl group, 4-methylphenyl and 3-trichloromethylphenyl group.

$R^1$ is a straight chain, branched chain or cyclic, saturated or unsaturated hydrocarbon group having 1 to 6 carbon atoms, which may have a substituent such as an alkoxyl group having 1 to 6 carbon atoms, a thioalkoxyl group, a mono- or di-substituted amino group, a halogen, a cyano group, a nitro group, a nitrato group, a hydroxyl group, a phenoxy group, a phenylthio group, a piperidino group, a pyrrolidino group, a substituted piperazinyl group, a morpholino group and a phenyl group. The phenyl substituent may further have one or two substituents selected from the group consisting of a halogen, a cyano group, a trihalomethyl group, an azido group, a disubstituted amino group, a lower alkyl group having 1 to 4 carbon atoms, a lower alkoxy group having 1 to 4 carbon atoms, a lower alkylthio group, a lower alkylcarbonyl group, a lower alkoxycarbonyl group, a carbamoyl group, a halomethyl group and a hydroxymethyl group. As the hydrocarbon group containing oxygen represented by $R^1$, an alkoxyalkyl group is preferable, and as the substituent of the hydrocarbon group represented by $R^1$, a disubstituted amino group is preferable for use in the present invention.

The nitrogen atom contained in the hydrocarbon group represented by $R^1$ may link with a group selected from the group consisting of a lower alkyl group having 1 to 3 carbon atoms, an aryl group or an aralkyl group.

Further, $R^1$ may be a group represented by $-A-Ar^2-(B)_n-Ar^3$, in which A, $Ar^2$, $Ar^3$, B and n are respectively the same as those defined previously in the general formula of the 1,4-dihydropyridine derivatives.

Preferred examples of the group represented by $R^1$ are a methyl group, an ethyl group, a propyl group, a n-butyl group, a n-pentyl group, a n-hexyl group, an isopropyl group, an isobutyl group, a cyclopentyl group, a cyclohexyl group, a propenyl group, a 2-butenyl group, a 3-butenyl group, a 2-pentynyl group, a 2,4-hexadienyl group, a 2,4-hexadiynyl group, a hexa-2-yn-4-ene group and a hexa-2-en-4-yne group.

Preferred examples of the group represented by $R^1$ containing therein an oxygen atom, a sulfur atom or a nitrogen atom are a methoxyethyl group, an ethoxyethyl group, a methoxypropyl group, an ethoxypropyl group, a methylthioethyl group, an ethylthioethyl group, a methylthiopropyl group, an ethylthiopropyl group, a phenylthioethyl group, a 2-(N-methylamino)ethyl group, a 2-(N,N-dimethylamino)ethyl group, a 2-(N-phenyl-N-methylamino)ethyl group, a 2-(N-benzyl-N-methylamino)ethyl group, a 2-(4-phenylpiperazin-1-yl)-ethyl group, a 2-{4-(diphenylmethyl)-piperazinyl}ethyl group, a 2-morpholinoethyl group, a N-benzylpyrrolidin-3-yl group and an N-benzylpiperidin- 3-yl group.

A represents (i) a straight chain or branched chain unsaturated hydrocarbon group, preferably a straight

chain or branched hydrocarbon having 3 to 12 carbon atoms, (ii) a cyclic unsaturated hydrocarbon group, preferably a cyclic unsaturated hydrocarbon having 4 to 12 carbon atoms, preferably in which unsaturated hydrocarbon groups, at least an unsaturated bond thereof is conjugated with the aromatic hydrocarbon or heterocyclic group represented by $Ar^2$, or (iii) a group selected from the group consisting of -R-O-N=CH-, -R-N=N-, -R-CH=N- and -R-N=CH-, in which R is an alkylene group having 1 to 6 carbon atoms.

The hydocarbon groups represented by A may have a substituent selected from the group consisting of a halogen, a phenyl group, a pyridyl group, a thienyl group, a furyl group, a cyano group, an alkylcarbonyl group and an alkoxycarbonyl group.

Preferred examples of the group represented by A are as follows:

$-CH_2-CH{=}CH-$ , $-CH_2(CH{=}CH)_2$ ,

$-CH_2-C{\equiv}C-$ , $-CH_2(C{\equiv}C)_2$ , $-CH_2(CH{=}CH)_3$ ,

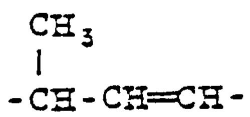 , 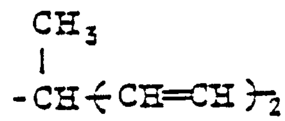 , 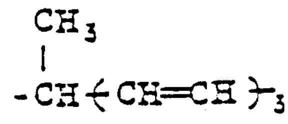 ,

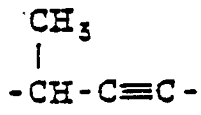 , 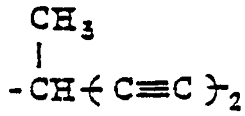 , 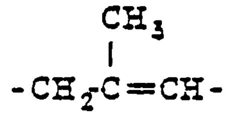 ,

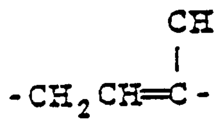 , 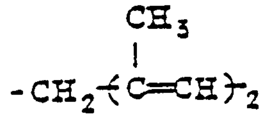 , 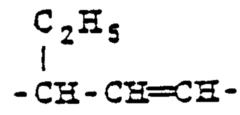 ,

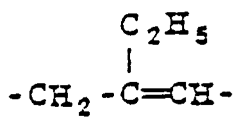 , 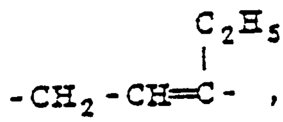 , 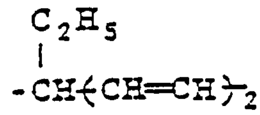 ,

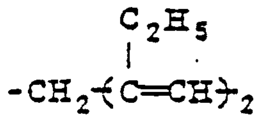 , $-CH_2-CF{=}CH-$ , 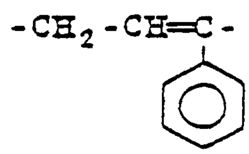 ,

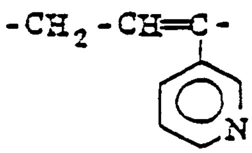 , $-CH_2-CH{=}CH-C{\equiv}C-$ ,

$-CH_2CH_2-CH{=}CH-$ , $-CH_2CH_2-O-N{=}CH-$ ,

$-CH_2CH_2-N{=}CH-$ , $-CH_2CH_2CH{=}N-$ , $-CH_2CH_2-N{=}N-$ , and

$-CH_2-C{\equiv}C-CH{=}CH-$ .

$Ar^2$ may be o-, m- or p-phenylene group, a furandiyl group, a thiophenediyl group, a pyridinediyl group or a pyrrolediyl group, or $Ar^2$ may be linked with A by a methylene group, an ethylene group, a vinyl group, an oxygen atom, a sulfur atom or an amino group to form a 5- or 6-membered condensed ring.

Preferred examples of the group represented by $Ar^2$ are as follows:

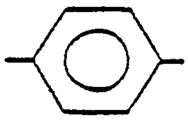 , 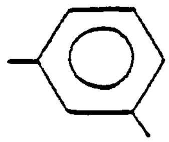 , 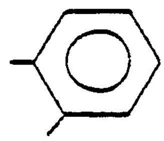 , 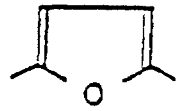 ,

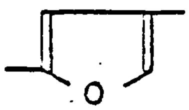 , 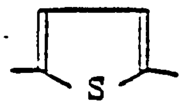 , 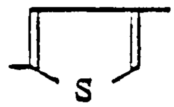 , 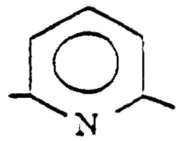 ,

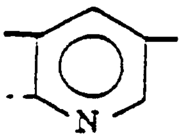 , 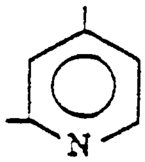 , 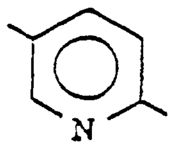 , 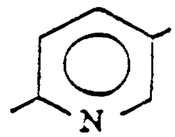 ,

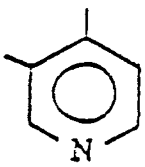 , 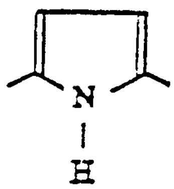 , 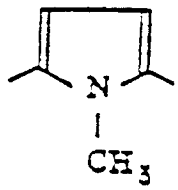 ,

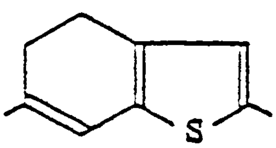 , 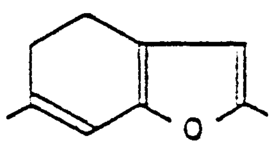 , 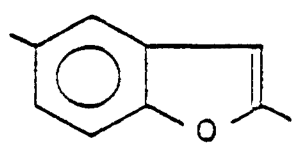 ,

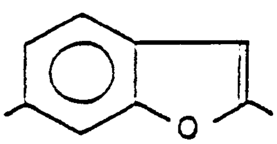 , 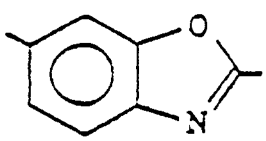 , 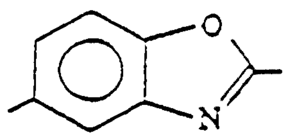 ,

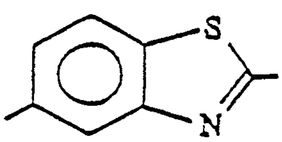 and 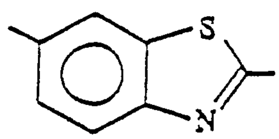 .

B may be an alkylene or alkenylene group having 1 to 3 carbon atoms, which may have a substituent selected from the group consisting of a lower alkyl group having 1 to 4 carbon atoms, a cyclic alkyl group, an aryl group and an aralkyl group.

Preferred examples of the group represented by B are a methylene group, an ethylene group, a trimethylene group, an ethylidene group, a benzylidene group, a vinylene group, a cyclohexylmethylene group and a 2-phenylethylidene group.

$Ar^3$ may be a heterocyclic group with five or six members containing therein at least one atom selected from the group consisting of nitrogen, oxygen and sulfur. Preferred examples of the group represented by $Ar^3$ are a 2-pyrrolyl group, a 2-pyridyl group, a 3-pyridyl group, a 4-pyridyl group, an imidazolyl group, a 2-oxazolyl group, a 4-oxazolyl group, a 2-thiazolyl group, a triazolyl group and a tetrazolyl group.

B may be linked with $Ar^2$ or $Ar^3$ by a methylene group, an ethylene group, a vinylene group, an oxygen atom, a sulfur atom or a nitrogen atom to form a ring with five or six members, such as

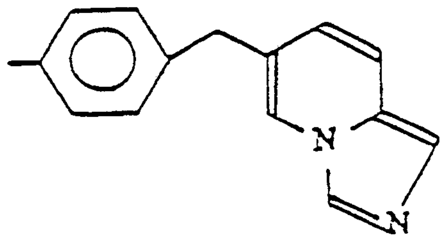
,
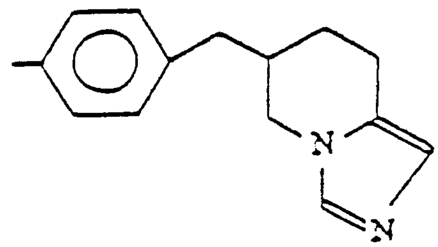
,

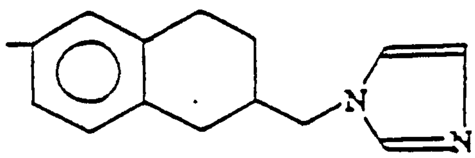
and
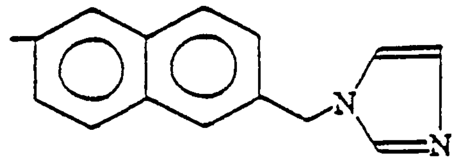
.

Each of the Optical active 1,4-dihydropyridine derivatives having the following formula (I) according to the present invention has a geometrical isomerism E or Z and an optical rotation (+) or (-).

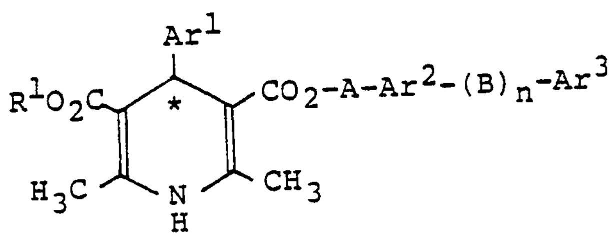
(I)

* optically active cite

Specific examples of the optical active 1,4-dihydropyridine derivative are as follows:

(+) or (-)-3-[4-(1-imidazolylmethyl)phenyl]-2-propen-1-yl methyl 1,4 dihydro-2,6-dimethyl-4-phenylpyridine-3,5-dicarboxylate,

(+) or (-)-3-[4-(1-imidazolylmethyl)phenyl]-2-propen-1-yl methyl 4-(3-azidophenyl)-1,4-dihydro-2,6-dimethylpyridine-3,- 5-dicarboxylate,

(+) or (-)-3-[4-(1-imidazolylmethyl)phenyl]-2-propen-1-yl methyl 1,4-dihydro-2,6-dimethyl-4-(3-trichloromethylphenyl)pyridine 3,5-dicarboxylate,

(+) or (-)-3-[4-(1-imidazolylmethyl)phenyl]-2-propen-1-yl methyl 4-(3-cyanophenyl)-1,4-dihydro-2,6-dimethylpyridine-3,-5-dicarboxylate,

(+) or (-)-3-[4-(1-imidazolylmethyl)phenyl]-2-propen-1-yl methyl 1,4-dihydro-2,6-dimethyl-4-(3-methylphenyl)pyridine-3,-5-dicarboxylate,

(+) or (-)-3-[4-(1-imidazolylmethyl)phenyl]-2-propen-1-yl methyl 1,4-dihydro-2,6-dimethyl-4-(3-methoxyphenyl)pyridine-3,5-dicarboxylate,

(+) or (-)-3-[4-(1-imidazolylmethyl)phenyl]-2-propen-1-yl methyl 1,4-dihydro-2,6-dimethyl-4-(4-methythiophenyl)pyridine-3,5-dicarboxylate,

(+) or (-)-3-[4-(1-imidazolylmethyl)phenyl]-2-propen-1-yl methyl 1,4-dihydro-2,6-dimethyl-4-(3-hydroxyphenyl)pyridine-3,5-dicarboxylate,

(+) or (-)-3-[4-(1-imidazolylmethyl)phenyl]-2-propen-1-yl methyl 4-(3-benzyloxyphenyl)-1,4-dihydro-2,6-dimethylpyridine3,5-dicarboxylate,

(+) or (-)-3-[4-(1-imidazolylmethyl)phenyl]-2-propen-1-yl methyl 4-(3-cinnamyloxyphenyl)-1,4-dihydro-2,6-dimethylpyridine-3,5-dicarboxylate,
(+) or (-)-3-[4-(1-imidazolylmethyl)phenyl]-2-propen-1-yl methyl 4-(3-benzoylphenyl)-1,4-dihydro-2,6-dimethylpyridine-3,5-dicarboxylate,
(+) or (-)-3-[4-(1-imidazolylmethyl)phenyl]-2-propen-1-yl methyl 4-(3-chlorophenyl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(3-fluorophenyl)-pyridine-3,5-dicarboxylate,
(+) or (-)-3-[4-(1-imidazolylmethyl)phenyl]-2-propen-1-yl methyl 1,4-dihydro-2,6-dimethyl-4-(2-nitrophenyl)-pyridi ne-3,5-dicarboxylate,
(+) or (-)-3-[4-(1-imidazolylmethyl)phenyl]-2-propen-1-yl methyl 1,4-dihydro-2,6-dimethyl-4-(3-nitrophenyl)-pyridine-3,5-dicarboxylate,
(+) or (-)-3-[4-(1-imidazolylmethyl)phenyl]-2-propen-1-yl methyl 1,4-dihydro-2,6-dimethyl-4-(4-nitrophenyl)-pyridine-3,5-dicarboxylate,
(+) or (-)-3-[4-(1-imidazolylmethyl)phenyl]-2-propen-1-yl methyl 4-(3-bromophenyl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(3-iodophenyl)-pyridine-3,5-dicarboxylate,
(+) or (-)-3-[4-(1-imidazolylmethyl)phenyl]-2-propen-1-yl methyl 4-(2-chloro-3-nitrophenyl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(2-fluoro-3-nitrophenyl)pyridine-3,5-dicarboxylate,
(+) or (-)-3-[4-(1-imidazolylmethyl)phenyl]-2-propen-1-yl methyl 4-(3-chloro-2-fluorophenyl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(2,3-difluorophenyl)pyridine-3,5-dicarboxylate,
(+) or (-)-3-[4-(1-imidazolylmethyl)phenyl]-2-propen-1-yl methyl 1,4-dihydro-2,6-dimethyl-4-(2-furyl)pyridine-3,5-dicarboxylate,
(+) or (-)-3-[4-(1-imidazolylmethyl)phenyl]-2-propen-1-yl methyl 1,4-dihydro-2,6-dimethyl-4-(3-furyl)pyridine-3,5-dicarboxylate,
(+) or (-)-3-[4-(1-imidazolylmethyl)phenyl]-2-propen-1-yl methyl 1,4-dihydro-2,6-dimethyl-4-(2-thienyl)-pyridine-3,5-dicarboxylate,
(+) or (-)-3-[4-(1-imidazolylmethyl)phenyl]-2-propen-1-yl methyl 1,4-dihydro-2,6-dimethyl-4-(3-thienyl)-pyridine-3,5- dicarboxylate,
(+) or (-)-3-[4-(1-imidazolylmethyl)phenyl]-2-propen-1-yl methyl 1,4-dihydro-2,6-dimethyl-4-(1-naphthyl)-pyridine-3,5-dicarboxylate,
(+) or (-)-3-[4-(1-imidazolylmethyl)phenyl]-2-propen-1-yl methyl 1,4-dihydro-2,6-dimethyl-4-(1-isoquinolyl)-pyridine-3,-5-dicarboxylate,
(+) or (-)-3-[4-(1-imidazolylmethyl)phenyl]-2-propen-1-yl methyl 1,4-dihydro-2,6-dimethyl-4-(2-quinoxalyl)-pyridine-3,-5-dicarboxylate,
(+) or (-)-3-[4-(1-imidazolylmethyl)phenyl]-2-propen-1-yl methyl 4-(2-chloropyridyl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(2-methylpyridyl)pyridine-3,5-dicarboxylate,
(+) or (-)-3-[4-(1-imidazolylmethyl)phenyl]-2-propen-1-yl methyl 1,4-dihydro-2,6-dimethyl-4-(2-methoxypyridyl)pyridine-3,5-dicarboxylate,
(+) or (-)-3-[4-(1-imidazolylmethyl)phenyl]-2-propen-1-yl methyl 4-(5-bromopyridyl)-1,4-dihydro-2,6-dimethylpyridine-3,5-dicarboxylate,
(+) or (-)-3-[4-(1-imidazolylmethyl)phenyl]-2-propen-1-yl methyl 4-(2-aminopyridyl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(2-mercaptopyridyl)pyridine-3,5-dicarboxylate,
(+) or (-)-3-[4-(1-imidazolylmethyl)phenyl]-2-propen-1-yl methyl 1,4-dihydro-2,6-dimethyl-4-(3-pyridazinyl)-pyridine-3,5-dicarboxylate,
(+) or (-)-3-[4-(1-imidazolylmethyl)phenyl]-2-propen-1-yl methyl 1,4-dihydro-2,6-dimethyl-4-pyrazinylpyridine-3,5-dicarboxylate,
(+) or (-)-3-[4-(1-imidazolylmethyl)phenyl]-2-propen-1-yl methyl 1,4-dihydro-2,6-dimethyl-4-(2-pyrimidyl)-pyridine-3,5- dicarboxylate,

(+) or (-)-3-[4-(1-imidazolylmethyl)phenyl]-2-propen-1-yl methyl 4-(7-benzoxazolyl)-1,4-dihydro-2,6-dimethylpyridine-3,5-dicarboxylate,
(+) or (-)-3-[4-(1-imidazolylmethyl)phenyl]-2-propen-1-yl methyl 4-(7-benzothiazolyl)-1,4-dihydro-2,6-dimethylpyridine-3,5-dicarboxylate,
(+) or (-)-3-[4-(1-imidazolylmethyl)phenyl]-2-propen-1-yl methyl-4-(2,1,3-benzoxadiazole-3-yl)-1,4-dihydro-2,6-dimethylpyridine-3,5-dicarboxylate,
(+) or (-)-3-[4-(1-imidazolylmethyl)phenyl]-2-propen-1-yl methyl 4-(2,1,3-benzothiadiazole-3-yl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(3-indolyl)-pyridine-3,5-dicarboxylate,
(+) or (-)-3-[4-(1-imidazolylmethyl)phenyl]-2-propen-1-yl methyl 1,4-dihydro-2,6-dimethyl-4-(2,3,4-trimethoxyphenyl)pyridine-3,5-dicarboxylate,
(+) or (-)-3-[4-(1-imidazolylmethyl)phenyl]-2-propen-1-yl methyl 1,4-dihydro-2,6-dimethyl-4-(2,3,4-trifluorophenyl)pyridine-3,5-dicarboxylate,
(+) or (-)-3-[4-(1-imidazolylmethyl)phenyl]-2-propen-1-yl methyl 1,4-dihydro-2,6-dimethyl-4-(2,3,6-trichlorophenyl)pyridine-3,5-dicarboxylate,
(+) or (-)-3-[4-(1-imidazolylmethyl)phenyl]-2-propen-1-yl methyl 1,4-dihydro-2,6-dimethyl-4-(2,4,6-trimethylphenyl)pyridine-3,5-dicarboxylate,
(+) or (-)-3-[4-(1-imidazolylmethyl)phenyl]-2-propen-1-yl methyl 1,4-dihydro-2,6-dimethyl-4-(3,4,5-triethoxyphenyl)pyridine-3,5-dicarboxylate,
(+) or (-)-3-[4-(1-imidazolylmethyl)phenyl]-2-propen-1-yl methyl 4-(2-chloro-3,5-dinitrophenyl)-1,4-dihydro-2,6-dimethylpyridine-3,5-dicarboxylate,
(+) or (-)-3-[4-(1-imidazolylmethyl)phenyl]-2-propen-1-yl methyl 1,4-dihydro-2,6-dimethyl-4-(2-cyanophenyl)-pyridine- 3,5-dicarboxylate,
(+) or (-)-3-[4-(1-imidazolylmethyl)phynyl]-2-propen-1-yl methyl 1,4-dihydro-2,6-dimethyl-4-(3-trifluoromethylphenyl) pyridine-3,5-dicarboxylate,
(+) or (-)-3-[4-(1-imidazolylmethyl)phenyl]-2-propen-1-yl methyl 1,4-dihydro-2,6-dimethyl-4-(2,3-dichlorophenyl) pyridine-3,5-dicarboxylate,
(+) or (-)-3-[4-(1-imdazolylmethyl)phenyl]-2-propen-1-yl methyl 1,4-dihydro-2,6-dimethyl-4-(4-quinolyl)-pyridine-3,5-dicarboxylate,
(+) or (-)-3-[4-(1-imidazolylmethyl)phenyl]-2-propen-1-yl mehyl 1,4-dihydro-2,6-dimethyl-4-(3-pyridyl)-pyridine-3,5-dicarboxylate,
(+) or (-) methyl 3-[4-(1,2,3,4-tetrazol-1-ylmethyl)phenyl]-2-propen-1-yl 1,4-dihydro-2,6-dimethyl-4-(3-nitrophenyl) pyridine-3,5-dicarboxylate,
(+) or (-) methyl 3-[4-(1,2,4-triazol-1-ylmethyl)phenyl]-2-propen-1-yl 1,4-dihydro-2,6-dimethyl-4-(3-nitrophenyl) pyridine-3,5-dicarboxylate,
(+) or (-)-3-[4-(1,3,4-triazol-1-ylmethyl)phenyl]-2-propen-1-yl 1,4-dihydro-2,6-dimethyl-4-(3-nitrophenyl)-pyridine-3,5-dicarboxylate,
(+) or (-) methyl 3-[4-(3-pyridylmethyl)phenyl]-2-propen-1-yl 1,4-dihydro-2,6-dimethyl-4-(3-nitrophenyl)-pyridine-3,5-dicarboxylate,
(+) or (-)-3-[4-(1-imidazolyl)phenyl]-2-propen-1-ylmethyl 1,4-dihydro-2,6-dimethyl-4-(3-nitrophenyl)pyridine-3,5-dicarboxylate,
(+) or (-)-3-[6-(1-imidazolylmethyl)pyridine-2-yl]-2-propen-1-yl methyl 1,4-dihydro-2,6-dimethyl-4-(3-nitrophenyl)pyridine-3,5-dicarboxylate,
(+) or (-)-3-[3-(1-imidazolylmethyl)phenyl]-2-propen-1-yl methyl 1,4-dihydro-2,6-dimethyl-4-(3-nitrophenyl)-pyridine-3,5-dicarboxylate,
(+) or (-)-3-[2-(1-imidzolylmethyl)phenyl]-2-propen-1-yl methyl 1,4-dihydro-2,6-dimethyl-4-(3-nitrophenyl)-pyridine-3,5-dicarboxylate,
(+) or (-)-3-[5-(1-imidazolylmethyl)-2-thiophene]-2-propen 1-yl methyl-1,4-dihydro-2,6-dimethyl-4-(3-nitrophenyl)pyridine-3,5-dicaboxylate,
(+) or (-)-3-[5-(1-imidzolylmethyl)-2-furan]-2-propen-1-yl methyl 1,4-dihydro-2,6-dimethyl-4-[3-nitrophenyl)-pyridine-3,5-dicarboxylate,
(+) or (-)-6-(1-imidzolylmethyl)benzoxazol-2-yl-methyl methyl 1,4-dihydro-2,6-dimethyl-4(3-nitrophenyl)-pyridine-3,5-dicarboxylate,
(+) or (-)-isopropyl 3-[4-(1-imidazolylmethyl)phenyl]-2-propen-1-yl 1,4-dihydro-2,6-dimethyl-4-(3-nitrophenyl) pyridine-3,5-di-carboxylate,
(+) or (-)-di-3-[4-(1-imidazolylmethyl)phenyl]-2-propen-1-yl 1,4-dihydro-2,6-dimethyl-4-{3-nitrophenyl)-pyridine-3,5-dicarboxylate,

(+) or (-)-5-[4-(1-imidazolylmethyl)phenyl]-2,4-pentadien-1-yl methyl-1,4-dihydro-2,6-dimethyl-4-(3-nitrophenyl)pyridine-3,5-dicarboxylate,
(+) or (-)-3-[4-(1-imidazolylmethyl)phenyl]-2-propen-1-yl 2-methoxyethyl 1,4-dihydro-2,6-dimethyl-4-(3-nitrophenyl) pyridine-3,5-dicarboxylate,
(+) or (-)-2,4-hexadien-1-yl 3-[4-(1-imidazolylmethyl)phenyl] 2-propen-1-yl 1,4-dihydro-2,6-dimethyl-4-(3-nitrophenyl) pyridine-3,5-dicarboxylate,
(+) or (-) cinnamyl 3-[4-(1-imidazolylmethyl)phenyl]-2-propen-1-yl 1,4-dihydro-2,6-dimethyl-4-(3-nitrophenyl)pyridine-3,5-dicarboxylate,
(+) or (-) ethyl 3-[4-(1-imidazolylmethyl)phenyl]-2-propen-1-yl 1,4-dihydro-2,6-dimethyl-4-(3-nitrophenyl)-pyridine-3,5-dicarboxylate,
(+) or (-)-5-[4-(1-imidazolylmethyl)phenyl]-pent-3-en-4-yne-1-yl methyl 1,4-dihydro-2,6-dimethyl-4-(3-nitrophenyl)pyridine-3,5-dicarboxylate,
(+) or (-)-3-[4-(1-imidazolylmethyl)phenyl]-2-propyne-1-yl methyl 1,4-dihydro-2,6-dimethyl-4-(3-nitrophenyl)-pyridine-3,5-dicarboxylate,
(+) or (-)-4-[4-(1-imidazolylmethyl)phenyl]-3-buten-2-yl methyl 1,4-dihydro-2,6-dimethyl-4-(3-nitrophenyl)-pyridine-3,5-dicarboxylate,
(+) or (-)-4-[4-(1-imidazolylmethyl)phenyl]-3-buten-1-yl methyl 1,4-dihydro-2,6-dimethyl-4-(3-nitrophenyl)-pyridine-3,5-dicarboxylate,
(+) or (-)-3-[4-(1-{1-imidazolyl)ethyl}phenyl]-2-propen-1-yl methyl 1,4-dihydro-2,6-dimethyl-4-(3-nitrophenyl)pyridine 3,5-dicarboxylate,
(+) or (-)-3-[4-(1-imidazolylmethyl)phenyl]-2-fluoro-2-propen-1-yl methyl 1,4-dihydro-2,6-dimethyl-4-(3-nitrophenyl) pyridine-3,5-dicarboxylate,
(+) or
(-)-3-[4-(1-imidazolylmethyl)phenyl]-2-methyl-2-propen-1-yl methyl 1,4-dihydro-2,6-dimethyl-4-[3-nitrophenyl)pyridine-3,5-dicarboxylate,
(+) or (-)-3-[4-(1-imidazolylmethyl)phenyl]-2-buten-1-yl methyl 1,4-dihydro-2,6-dimethyl-4-(3-nitrophenyl)-pyridine-3,5-dicarboxylate,
(+) or (-)-2-[4-(1-imidazolylmethyl)benzylideneamino]ethyl methyl 1,4-dihydro-2,6-dimethyl-4-(3-nitrophenyl)pyridine-3,5-dicarboxylate, and
(+) or (-)-2-[{4-(1-imidazolylmethyl)benylideneamino}oxy]ethylmethyl-1,4-dihydro-2,6-dimethyl-4-(3-nitrophenyl)pyridine-3, 5-dicarboxylate.

The optical active 1,4-dihydropyridine derivative of formula (I) can be prepared by either Process A or Process B as shown below:

[Process A]

In this process, the optical active 1,4-dihydropyridine derivative of formula (I) is prepared by allowing an optical active carboxylic acid derivative having formula (I-1) to react with an alcohol derivative having formula (I-2):

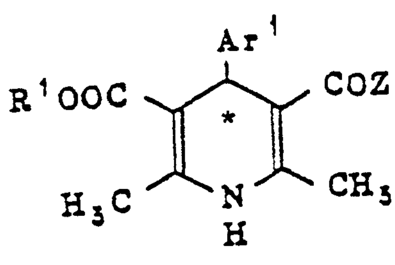

(I-1)

* indicates an optically active cite.

wherein $Ar^1$ represents an aromatic hydrocarbon group or an aromatic heteromonocyclic or heterobicyclic group containing therein 1 to 3 atoms selected from the group consisting of oxygen, sulfur and nitrogen; $R^1$ represents a hydrocarbon group which may have one or more substituents, including $-A-Ar^2-B-Ar^3$, in which A, B, $Ar^2$, and $Ar^3$ respectively the same as defined previously in formula (I), and Z represents a hydroxyl group, a halogen atom, a methyl-sulfonyloxy group or a benzotriazole-1-oxy group.

$HO-R^2$ (I-2)

wherein $R^2$ represents -A-$Ar^2$-B-$Ar^3$ as represented by $R^1$ when $R^1$ is a hydrocarbon group other than -A-$Ar^2$-B-$Ar^3$; or when $R^1$ is -A-$Ar^2$-B-$Ar^3$, $R^2$ is a hydrocarbon group other than -A-$Ar^2$-B-$Ar^3$.

In this process, optical active carboxylic acid derivatives of formula (I-1) in which Z is a hydroxyl group (hereinafter referred to as the carboxylic acid compounds) can be prepared by the methods as described in Japanese Laid-Open Patent Applications No. 63-20857 and No. 55-6457.

Optical active carboxylic acid derivatives of formula (I-1) in which Z is a halogen (hereinafter referred to as the halogen-substituted carboxylic acid compounds) can be prepared by the halogenation of the above-mentioned carboxylic acid compounds by use of a thionyl halide such as thionyl chloride, or a phosphorous halide such as phosphorous trichloride, phosphorus pentachloride, or phosphorous tribromide.

Optical active carboxylic acid derivatives of formula (I-1) in which Z is an active ester radical (hereinafter referred to as the active ester compounds) can be prepared by allowing the carboxylic acid compounds to react with N-hydroxysuccinimide, N-hydroxyphthalimide, or p-nitrophenol in the presence of a condensing agent such as "DCC"(N,N′-dicyclo-hexylcarbodiimide). The active ester radical in the present invention means, for example, a succinimidoxy group, a phthalimidoxy group, and a p-nitrophenoxy group employed in the field of the so-called peptide synthesis.

Specific examples of the optical active carboxylic acid derivative having formula (I-1) for use in the present nvention are as follows:
(+)-1,4-dihydro-2,6-dimethyl-4-(3-nitrophenyl)pyridine-3,5-dicarboxylic acid monomethyl ester,
(-)-1,4-dihydro-2,6-dimethyl-4-(3-nitrophenyl)pyridine-3,5-dicarboxylic acid monomethyl ester,
(+)-1,4-dihydro-2,6-dimethyl-4-(3-nitrophenyl)pyridine-3,5-dicarboxylic acid mono(E)-3-[4-(1-imidazolylmethyl) phenyl]-2-propen-1-yl ester,
(-)-1,4-dihydro-2,6-dimethyl-4-(3-nitrophenyl)pyridine-3,5-dicarboxylic acid mono(E)-3-[4-(1-imidazolylmethyl) phenyl]-2-propen-1-yl ester,
(+)-1,4-dihydro-2,6-dimethyl-4-(3-nitrophenyl)pyridine-3,5-dicarboxylic acid mono(Z)-3-[4-(1-imidazolylmethyl) phenyl]-2-propen-1-yl ester,
(-)-1,4-dihydro-2,6-dimethyl-4-(3-nitrophenyl)pyridine-3,5-dicarboxylic acid mono(Z)-3-[4-(1-imidazolylmethyl) phenyl]-2-propen-1-yl ester,
(+)-1,4-dihydro-2,6-dimethyl-4-(3-nitrophenyl)pyridine-3,5-dicarboxylic acid monochloride monomethyl ester,
(-)-1,4-dihydro-2,6-dimethyl-4-(3-nitrophenyl)pyridine-3,5-dicarboxylic acid monochloride monomethyl ester,
(+)-1,4-dihydro-2,6-dimethyl-4-(3-nitrophenyl)pyridine-3,5-dicarboxylic acid monochloride mono(E)-3-[4-(1-imidazolylmethyl)phenyl]-2-propen-1-yl ester,
(-)-1,4-dihydro-2,6-dimethyl-4-(3-nitrophenyl)pyridine-3,5-dicarboxylic acid monochloride mono(E)-3-[4-(1-imidazolylmethyl)phenyl]-2-propen-1-yl ester,
(+)-1,4-dihydro-2,6-dimethyl-4-(3-nitrophenyl)pyridine-3,5-dicarboxylic acid monochloride mono(Z)-3-[4-(1-imidazolylmethyl)phenyl]-2-propen-1-yl ester,
(-)-1,4-dihydro-2,6-dimethyl-4-(3-nitrophenyl)pyridine-3,5-dicarboxylic acid monochloride mono(Z)-3-[4-(1-imidazolylmethyl)phenyl]-2-propen-1-yl ester,
(+)-methyl succinimide-N-yl 1,4-dihydro-2,6-dimethyl-4-(3-nitrophenyl)pyridine-3,5-dicarboxylate,
(-)-methyl succinimide-N-yl 1,4-dihydro-2,6-dimethyl-4-(3-nitrophenyl)pyridine-3,5-dicarboxylate,
(+)-(E)-3-[4-(1-imidazolylmethyl)phenyl]-2-propen-1-yl succinimide-N-yl 1,4-dihydro-2,6-dimethyl-4-(3-nitrophenyl)pyridine-3,5-dicarboxylate,
(-)-(E)-3-[4-(1-imidazolylmethyl)phenyl]-2-propen-1-yl succinimide-N-yl 1,4-dihydro-2,6-dimethyl-4-(3-nitrophenyl)pyridine-3,5-dicarboxylate,
(+)-(Z)-3-[4-(1-imidazolylmethyl)phenyl]-2-propen-1-yl succinimide-N-yl 1,4-diphydro-2,6-dimethyl-4-(3-nitrophenyl)pyridine-3,5-dicarboxylate,
(-)-(Z)-3-[4-(1-imidazolylmethyl)phenyl]-2-propen-1-yl succinimide-N-yl 1,4-diphydro-2,6-dimethyl-4-(3-nitrophenyl)pyridine-3,5-dicarboxylate,
(+)-1,4-dihydro-2,6-dimethyl-4-(3-nitrophenyl)pyridine-3,5-dicarboxylic acid monoethyl ester,
(-)-1,4-dihydro-2,6-dimethyl-4-(3-nitrophenyl)pyridine-3,5-dicarboxylic acid monoethyl ester,
(+)-1,4-dihydro-2,6-dimethyl-4-(3-nitrophenyl)pyridine-3,5-dicarboxylic acid mono-n-propyl ester,
(-)-1,4-dihydro-2,6-dimethyl-4-(3-nitrophenyl)pyridine-3,5-dicarboxylic acid mono-n-propyl ester,
(+)-1,4-dihydro-2,6-dimethyl-4-(3-nitrophenyl)pyridine-3,5-dicarboxylic acid mono-isopropyl ester,
(-)-1,4-dihydro-2,6-dimethyl-4-(3-nitrophenyl)pyridine-3,5-dicarboxylic acid mono-isopropyl ester,
(+)-1,4-dihydro-2,6-dimethyl-4-(3-nitrophenyl)pyridine-3,5-dicarboxylic acid mono-2-methoxyethyl ester,
(-)-1,4-dihydro-2,6-dimethyl-4-(3-nitrophenyl)pyridine-3,5-dicarboxylic acid mono-2-methoxyethyl ester,
(+)-1,4-dihydro-2,6-dimethyl-4-(3-nitrophenyl)pyridine-3,5-dicarboxylic acid mono-2,4-hexadienyl ester,
(-)-1,4-dihydro-2,6-dimethyl-4-(3-nitrophenyl)pyridine-3,5-dicarboxylic acid mono-2,4-hexadienyl ester,

(+)-1,4-dihydro-2,6-dimethyl-4-(3-nitrophenyl)pyridine-3,5-dicarboxylic acid mono-3-phenyl-2-propen-1-yl ester,
(-)-1,4-dihydro-2,6-dimethyl-4-(3-nitrophenyl)pyridine-3,5-dicarboxylic acid mono-3-phenyl-2-propen-1-yl ester,
(+)-1,4-dihydro-2,6-dimethyl-4-(2-nitrophenyl)pyridine-3,5-dicarboxylic acid monomethyl ester,
(-)-1,4-dihydro-2,6-dimethyl-4-(2-nitrophenyl)pyridine-3,5-dicarboxylic acid monomethyl ester,
(+)-1,4-dihydro-2,6-dimethyl-4-(4-nitrophenyl)pyridine-3,5-dicarboxylic acid monomethyl ester,
(-)-1,4-dihydro-2,6-dimethyl-4-(4-nitrophenyl)pyridine-3,5-dicarboxylic acid monomethyl ester,
(+)-1,4-dihydro-2,6-dimethyl-4-(3-trifluorophenyl)pyridine-3,5-dicarboxylic acid monomethyl ester,
(-)-1,4-dihydro-2,6-dimethyl-4-(3-trifluorophenyl)pyridine-3,5-dicarboxylic acid monomethyl ester,
(+)-1,4-dihydro-2,6-dimethyl-4-(4-methoxyphenyl)pyridine-3, 5-dicarboxylic acid monomethyl ester,
(-)-1,4-dihydro-2,6-dimethyl-4-(4-methoxyphenyl)pyridine-3, 5-dicarboxylic acid monomethyl ester,
(+)-1,4-dihydro-2,6-dimethyl-4-pyridylpyridine-3,5-dicarboxylic acid monomethyl ester,
(-)-1,4-dihydro-2,6-dimethyl-4-pyridiylpyridine-3,5-dicarboxylic acid monomethyl ester,
(+)-1,4-dihydro-2,6-dimethyl-4-furylpyridine-3,5-dicarboxylic acid monomethyl ester,
(-)-1,4-dihydro-2,6-dimethyl-4-furylpyridine-3,5-dicarboxylic acid monomethyl ester,
(+)-1,4-dihydro-2,6-dimethyl-4-thienylpyridine-3,5-dicarboxylic acid monomethyl ester,
(-)-1,4-dihydro-2,6-dimethyl-4-thienylpyridine-3,5-dicarboxylic acid monomethyl ester,
(+)-1,4-dihydro-2,6-dimethyl-4-(2-thienyl)pyridine-3,5-dicarboxylic acid monomethyl ester,
(-)-1,4-dihydro-2,6-dimethyl-4-(2-thienyl)pyridine-3,5-dicarboxylic acid monomethyl ester,
(+)-1,4-dihydro-2,6-dimethyl-4-(1-naphthyl)pyridine-3,5-dicarboxylic acid monomethyl ester,
(-)-1,4-dihydro-2,6-dimethyl-4-(1-naphthyl)pyridine-3,5-dicarboxylic acid monomethyl ester,
(+)-1,4-dihydro-2,6-dimethyl-4-(4-quinolyl)pyridine-3,5-dicarboxylic acid monomethyl ester,
(-)-1,4-dihydro-2,6-dimethyl-4-(4-quinolyl)pyridine-3,5-dicarboxylic acid monomethyl ester,
(+)-4-(2-cyanophenyl)-1,4-dihydro-2,6-dimethylpyridine-3,5-dicarboxylic acid monomethyl ester,
(-)-4-(2-cyanophenyl)-1,4-dihydro-2,6-dimethylpyridine-3,5-dicarboxylic acid monomethyl ester,
(+)-4-(2,3-dichlorophenyl)-1,4-dihydro-2,6-dimethylpyridine-3,5-dicarboxylic acid monomethyl ester,
(-)-4-(2,3-dichlorophenyl)-1,4-dihydro-2,6-dimethylpyridine-3,5-dicarboxylic acid monomethyl ester,
(+)-1,4-dihydro-2,6-dimethyl-4-(3-trichloromethylphenyl) pyridine-3,5-dicarboxylic acid monomethyl ester,
(-)-1,4-dihydro-2,6-dimethyl-4-(3-trichloromethylphenyl) pyridine-3,5-dicarboxylic acid monomethyl ester,
(+)-1,4-dihydro-2,6-dimethyl-4-(4-methylphenyl)pyridine-3,5-dicarboxylic acid monomethyl ester,
(-)-1,4-dihydro-2,6-dimethyl-4-(4-methylphenyl)pyridine-3,5-dicarboxylic acid monomethyl ester,
(+)-1,4-dihydro-2,6-dimethyl-4-(4-methylthiophenyl)pyridine-3,5-dicarboxylic acid monomethyl ester,
(-)-1,4-dihydro-2,6-dimethyl-4-(4-methylthiophenyl)pyridine-3,5-dicarboxylic acid monomethyl ester,
(+)-4-(3-azidophenyl)-1,4-dihydro-2,6-dimethylpyridine-3,5-dicarboxylic acid monomethyl ester, and
(-)-4-(3-azidophenyl)-1,4-dihydro-2,6-dimethylpyridine-3,5-dicarboxylic acid monomethyl ester.

Specific examples of the alcohol derivative of formula (I-2) for use in the present invention are methanol, ethanol, n-propanol, isopropanol, 2-methoxyethanol, cinnamyl alcohol, 3-[4-(1-imidazolylmethyl)-phenyl]-2-propen-1-ol, 3-[4-(3-pyridylmethyl)phenyl]-2-propen-1-ol, 3-[4-(1,2,4-triazole-1-yl-methyl)phenyl]-2-propen-1-ol, 3-[4-(1,2,3,4-tetrazole-1-yl-methyl)phenyl]-2-propen-1-ol, 3-[5-(1-imidazolylmethyl)furan]-2-propen-1-ol, and 5-[4-(1-imidazolylmethyl)furan]-2,4-pentadiene-1-ol.

It is preferable that the previously mentioned carboxylic acid compounds of formula (I-1) be allowed to react with the above-mentioned alcohol derivative of formula (I-2) in the presence of an acid or a condensing agent.

Examples of the acid are an inorganic acid such as HCl and $H_2SO_4$, and a Lewis acid such as $BF_3$. It is preferable that such acids be employed in an amount of 0.1 to 10 equivalents to the carboxylic acid derivative of formula (I-1).

As the condensing agent, for example, "DCC" can be employed. When such a condensing agent is employed, it is preferable that the amount of the condensing agent be 1 to 2 equivalents for the carboxylic acid derivative of fromula (I-1). When "DCC" is used as a condensing agent, a base such as N,N-dimethylaminopyridine may also be used, preferably in an amount of 0.1 to 1 equivalent to the "DCC".

The above reaction can be carried out in an inactive solvents such as benzene, toluene, dichloromethane, chloroform, acetonitrile, dichloroethane or acetone. In order to carry out the reaction smoothly, it is preferable that the reaction temperature be in the range of 0-120 °C.

The reaction of the halogen-substituted carboxylic acid compounds or the active ester compounds with the alcohol derivative of formula (I-2) can be carried out in inactive solvents, for example, aromatic hydrocarbons such as benzene and toluene, halogenated hydrocarbon such as chloroform and dichloromethane, amides such as dimethylformamide, and nitriles such as acetonitrile.

In order to carry out the above reaction smoothly, it is preferable that a base such as N,N-dimethylaminopyridine and trimethylamine be added to the reaction mixture, in an amount of 0.1 to 2 equivanlents to the carboxylic acid derivative. The reaction is usually carried out at -70 ° C to 100 ° C, preferably at -20 ° C to 50 ° C.

[Process B]

In this process, the optical active 1,4-dihydropyridine derivative of formula (I) is prepared by allowing a β ketocarboxylic acid ester derivative of formula (I-3) to react with an optical active β-aminoacrylic acid ester derivative of formula (I-4) and then by allowing the reaction product to react with an amine or an acid-added salt thereof.

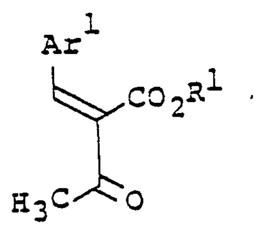

(I-3)

wherein $Ar^1$ and $R^1$ are respectively the same as those defined in formula (I-1).

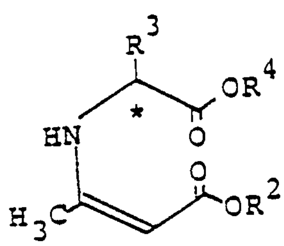

(I-4)

* optically active cite

wherein $R^2$ is the same as defined in formula (I-1), and $R^3$ and $R^4$ each represents an alkyl group having 1 to 6 carbon atoms, a phenyl group or a benzyl group.

The β ketocarboxylic acid ester derivative of formula (I-3) can be obtained by reacting an aromatic aldehyde derivative and a counterpart acetoacetic ester.

Specific example of the β ketocarboxylic acid ester derivative of formula (I-3) are:
methyl 2-(2-nitrobenzylidene)acetoacetate,
methyl 2-(3-nitrobenzylidene)acetoacetate,
methyl 2-(4-nitrobenzylidene)acetoacetate,
ethyl 2-(3-nitrobenzylidene)acetoacetate,
n-propyl 2-(3-nitrobenzylidene)acetoacetate,
isopropyl 2-(3-nitrobenzylidene)acetoacetate,
2-methoxyethyl 2-(3-nitrobenzylidene)acetoacetate,
2,4-hexadienyl 2-(3-nitrobenzylidene)acetoacetate,
3-phenyl-2-propen-1-yl 2-(3-nitrobenzylidene)acetoacetate,
3-[4-(1-imidazolylmethyl)phenyl]-2-propen-1-yl 2-(3-nitrobenzylidene)acetoacetate.
methyl 2-benzylideneacetate,
methyl 2-(2-cyanobenzylidene)acetoacetate,
methyl 2-(2,3-dicyclobenzylidene)acetoacetate,
methyl 2-(furylmethylene)acetoacetate,
methyl 2-(thienylmethylene)acetoacetate,
methyl 2-(naphthylmethylene)acetoacetate,
methyl 2-(quinolylmethylene)acetoacetate,

methyl 2-(3-azidobenzylidene)acetoacetate,
methyl 2-(3-trifuroromethylbenzylidene)acetoacetate,
methyl 2-(4-methoxybenzylidene)acetoacetate,
methyl 2-(4-methylbenzylidene)acetoacetate,
methyl 2-(4-methylthiobenzylidene)acetoacetate, and
methyl 2-(pyridylmethylene)acetoacetate.

In order to carry out the reaction smoothly, it is preferable to employ a catalyst. Examples of the catalyst include piperidine salts such as piperidine acetate. It is preferable that this reaction be carried out in solvents, for example, aromatic hydrocarbons such as benzene and toluene, nitriles such as acetonitrile, halogenated hydrocarbons such as dichloromethane and dichloroethane, alcohols such as methanol, ethanol and isopropyl alcohol, and ethers such as diisopropyl ether. The reaction is usually carried out at 0-100 °C, preferably at 10-70 °C.

The optical active β-aminoacrylic acid ester derivative of formula (I-4) can be obtained by mixing the corresponding acetoacetic ester and amino acid ester.

Specific examples of the optical active β-aminoacrylic acid ester derivative of formula (I-4) for use in the present invention are as follows:
methyl (S)-(+)-N-[(1-t-butoxycarbonyl-2-methyl)propyl]aminocrotonate,
methyl (R)-(-)-N-[(1-t-butoxycarbonyl-2-methyl)propyl]aminocrotonate,
methyl (S)-(+)-N-[(1-ethoxycarbonyl-2-methyl)propyl]aminocrotonate,
methyl (R)-(-)-N-[(1-ethoxycarbonyl-2-methyl)propyl]aminocrotonate,
methyl (S)-(+)-N-[(1-methoxycarbonyl-2-methyl)propyl]aminocrotonate,
methyl (R)-(-)-N-[(1-methoxycarbonyl-2-methyl)propyl]aminocrotonate,
methyl (S)-(+)-N-[(1-cyclohexyloxycarbonyl-2-methyl)propyl]aminocrotonate,
methyl (R)-(-)-N-[(1-cyclohexyloxycarbonyl-2-methyl)propyl]aminocrotonate,
methyl (S)-(+)-N-[(1-isopropyloxycarbonyl-2-methyl)propyl]aminocrotonate,
methyl (R)-(-)-N-[(1-isopropyloxycarbonyl-2-methyl)propyl]aminocrotonate,
methyl (S)-(+)-N-(1-t-butoxycarbonylethyl)aminocrotonate,
methyl (R)-(-)-N-(1-t-butoxycarbonylethyl)aminocrotonate,
methyl (S)-(+)-N-[(1-t-butoxycarbonyl-2,2-dimethyl)propyl]aminocrotonate,
methyl (R)-(-)-N-[(1-t-butoxycarbonyl-2,2-dimethyl)propyl]aminocrotonate,
methyl (S)-(+)-N-[(1-t-butoxycarbonylmethyl)butyl]aminocrotonate,
methyl (R)-(-)-N-[(1-t-butoxycarbonyl-3-methyl)butyl]aminocrotonate,
methyl (S)-(+)-N-[(1-t-butoxycarbonyl-1-phenyl)methyl]amino crotonate,
methyl (R)-(-)-N-[(1-t-butoxycarbonyl-1-phenyl)methyl]aminocrotonate,
methyl (S)-(+)-N-[(1-t-butoxycarbonyl-2-phenyl)ethyl]aminocrotonate,
methyl (R)-(-)-N-[(1-t-butoxycarbonyl-2-phenyl)ethyl]aminocrotonate,
ethyl (S)-(+)-N-[(1-t-butoxycarbonyl-2-methyl)propyl]aminocrotonate,
ethyl (R)-(-)-N-[(1-t-butoxycarbonyl-2-methyl)propyl]aminocrotonate,
n-propyl (S)-(+)-N-[(1-t-butoxycarbonyl-2-methyl)propyl]aminocrotonate,
n-propyl (R)-(-)-N-[(1-t-butoxycarbonyl-2-methyl)propyl]aminocrotonate,
isopropyl (S)-(+)-N-[(1-t-butoxycarbonyl-2-methyl)propyl]aminocrotonate,
isopropyl (R)-(-)-N-[(1-t-butoxycarbonyl-2-methyl)propyl]aminocrotonate,
2-methoxyethyl (S)-(+)-N-[(1-t-butoxycarbonyl-2-methyl)propyl]aminocrotonate,
2-methoxyethyl (R)-(-)-N-[(1-t-butoxycarbonyl-2-methyl)propyl]aminocrotonate,
3-phenyl-2-propen-1-yl (S)-(+)-N-[(1-t-butoxycarbonyl-2-methyl)propyl]aminocrotonate,
3-phenyl-2-propen-1-yl (R)-(-)-N-[(1-t-butoxycarbonyl-2-methyl)propyl]aminocrotonate,
(E)-3-[4-(1-imidazolylmethyl)phenyl]-2-propen-1-yl (S)-(+)-N-[(1-t-butoxycarbonyl-2-methyl)propyl]-aminocrotonate,
(E)-3-[4-(1-imidazolylmethyl)phenyl]-2-propen-1-yl (R)-(-)-N-[(1-t-butoxycarbonyl-2-methyl)propyl]-aminocrotonate,
(Z)-3-[4-(1-imidazolylmethyl)phenyl]-2-propen-1-yl (S)-(+)- N-[(1-t-butoxycarbonyl-2-methyl)propyl]-aminocrotonate,
(Z)-3-[4-(1-imidazolylmethyl)phenyl]-2-propen-1-yl (R)-(-)-N-[(1-t-butoxycarbonyl-2-methyl)propyl]-aminocrotonate,
3-[4-(1-imidazolylmethyl)phenyl]-2-propen-1-yl (S)-(+)-N-[(1-ethoxycarbonyl-2-methyl)propyl]-aminocrotonate,
3-[4-(1-imidazolylmethyl)phenyl]-2-propen-1-yl (R)-(-)-N-[(1-ethoxycarbonyl-2-methyl)propyl]aminocrotonate,
3-[4-(1-imidazolylmethyl)phenyl]-2-propen-1-yl (S)-(+)-N-[(1-cyclohexyloxycarbonyl-2-methyl)propyl]-

aminocrotonate,
3-[4-(1-imidazolylmethyl)phenyl]-2-propen-1-yl (R)-(-)-N-[(1-cyclohexyloxycarbonyl-2-methyl)propyl]-aminocrotonate,
3-[4-(1-imidazolylmethyl)phenyl]-2-propen-1-yl (S)-(+)-N-[(1-isopropyloxycarbonyl-2-methyl)propyl]-aminocrotonate,
3-[4-(1-imidazolylmethyl)phenyl]-2-propen-1-yl (R)-(-)-N-[(1-isopropyloxycarbonyl-2-methyl)propyl]-aminocrotonate,
3-[4-(1-imidazolylmethyl)phenyl]-2-propen-1-yl (S)-(+)-N-(1-t-butoxycarbonylethyl)aminocrotonate,
3-[4-(1-imidazolylmethyl)phenyl]-2-propen-1-yl (R)-(-)-N-(1-t-butoxycarbonylethyl)aminocrotonate,
3-[4-(1-imidazolylmethyl)phenyl]-2-propen-1-yl (S)-(+)-N-[(1-t-butoxycarbonyl-2,2-dimethyl)propyl]-aminocrotonate,
3-[4-(1-imidazolylmethyl)phenyl]-2-propen-1-yl (R)-(-)-N-[(1-t-butoxycarbonyl-2,2-dimethyl)propyl]-aminocrotonate,
3-[4-(1-imidazolylmethyl)phenyl]-2-propen-1-yl (S)-(+)-N-[(1-t-butoxycarbonyl-3-methyl)butyl]-aminocrotonate,
3-[4-(1-imidazolylmethyl)phenyl]-2-propen-1-yl (R)-(-)-N-[(1-t-butoxycarbonyl-3-methyl)butyl]aminocrotonate,
3-[4-(1-imidazolylmethyl)phenyl]-2-propen-1-yl (S)-(+)-N-[(1-t-butoxycarbonyl-1-phenyl)methyl]-aminocrotonate,
3-[4-(1-imidazolylmethyl)phenyl]-2-propen-1-yl (R)-(-)-N-[(1-t-butoxycarbonyl-1-phenyl)methyl]-aminocrotonate,
3-[4-(1-imidazolylmethyl)phenyl]-2-propen-1-yl (S)-(+)-N- [(1-t-butoxycarbonyl-2-phenyl)ethyl]-aminocrotonate,
3-[4-(1-imidazolylmethyl)phenyl]-2-propen-1-yl (R)-(-)-N-[(1-t-butoxycarbonyl-2-phenyl)ethyl]aminocrotonate,
3-[4-(3-pyridylmethyl)phenyl]-2-propen-1-yl (S)-(+)-N-[(1-t-butoxycarbonyl-2-methyl)propyl]aminocrotonate,
3-[4-(3-pyridylmethyl)phenyl]-2-propen-1-yl (R)-(-)-N-[(1-t-butoxycarbonyl-2-methyl)propyl]aminocrotonate,
3-[4-(1,2,4-triazole-1-yl-methyl)phenyl]-2-propen-1-yl (S)-(+)-N-[(1-t-butoxycarbonyl-2-methyl)propyl]-aminocrotonate,
3-[4-(1,2,4-triazole-1-yl-methyl)phenyl]-2-propen-1-yl (R)-(-)-N-[(1-t-butoxycarbonyl-2-methyl)propyl]-aminocrotonate,
3-[4-(1,2,3,4-tetrazole-1-yl-methyl)phenyl]-2-propen-1-yl (S)-(+)-N-[(1-t-butoxycarbonyl-2-methyl)propyl]-aminocrotonate,
3-[4-(1,2,3,4-tetrazole-1-yl-methyl)phenyl]-2-propen-1-yl (R)-(-)-N-[(1-t-butoxycarbonyl-2-methyl)propyl]-aminocrotonate,
3-[3-(1-imidazolylmethyl)phenyl]-2-propen-1-yl (S)-(+)-N-[(1-t-butoxycarbonyl-2-methyl)propyl]-aminocrotonate,
3-[3-(1-imidazolylmethyl)phenyl]-2-propen-1-yl (R)-(-)-N-[(1-t-butoxycarbonyl-2-methyl)propyl]-aminocrotonate,
3-[2-(1-imidazolylmethyl)phenyl]-2-propen-1-yl (S)-(+)-N-[(1-t-butoxycarbonyl-2-methyl)propyl]-aminocrotonate,
3-[2-(1-imidazolylmethyl)phenyl]-2-propen-1-yl (R)-(-)-N-[(1-t-butoxycarbonyl-2-methyl)propyl]-aminocrotonate,
3-[5-(1-imidazolylmethyl)furan]-2-propen-1-yl (S)-(+)-N-[(1-t-butoxycarbonyl-2-methyl)propyl]-aminocrotonate,
3-[5-(1-imidazolylmethyl)furan]-2-propen-1-yl (R)-(-)-N-[(1-t-butoxycarbonyl-2-methyl)propyl]aminocrotonate,
3-[5-(1-imidazolylmethyl)thiophene]-2-propen-1-yl (S)-(+)-N-[(1-t-butoxycarbonyl-2-methyl)propyl]-aminocrotonate,
3-[5-(1-imidazolylmethyl)thiophene]-2-propen-1-yl (R)-(-)-N-[(1-t-butoxycarbonyl-2-methyl)propyl]-aminocrotonate,
3-[2-(1-imidazolylmethyl)-1-methyl-pyrrole-5-yl]-2-propen-1-yl (S)-(+)-N-[(1-t-butoxycarbonyl-2-methyl)-propyl]aminocrotonate,
3-[2-(1-imidazolylmethyl)-1-methyl-pyrrole-5-yl]-2-propen-1-yl (R)-(-)-N-[(1-t-butoxycarbonyl-2-methyl)-propyl]aminocrotonate,
3-[4-[1-(1-imidazolyl)ethyl]phenyl]-2-propen-1-yl (S)-(+)-N-[(1-t-butoxycarbonyl-2-methyl)propyl]-aminocrotonate,
3-[4-[1-(1-imidazolyl)ethyl]phenyl]-2-propen-1-yl (R)-(-)-N-[(1-t-butoxycarbonyl-2-methyl)propyl]-aminocrotonate,
3-[4-(1-imidazolyl)phenyl]-2-propen-1-yl (S)-(+)-N-[(1-t-butoxycarbonyl-2-methyl)propyl]aminocrotonate,
3-[4-(1-imidazolyl)phenyl]-2-propen-1-yl (R)-(-)-N-[(1-t-butoxycarbonyl-2-methyl)propyl]aminocrotonate,

3-[4-(1-imidazolylmethyl)phenyl]-2-propen-1-yl (S)-(+)-N-[(1-t-butoxycarbonyl-2-methyl)propyl]-aminocrotonate,
3-[4-(1-imidazolylmethyl)phenyl]-2-propen-1-yl (R)-(-)-N-[(1-t-butoxycarbonyl-2-methyl)propyl]-aminocrotonate,
5-[4-(1-imidazolylmethyl)phenyl]-2,4-pentadiene-1-yl (S)-(+)-N-[(1-t-butoxycarbonyl-2-methyl)propyl]-aminocrotonate,
5-[4-(1-imidazolylmethyl)phenyl]-2,4-pentadiene-1-yl (R)-(-)-N-[(1-t-butoxycarbonyl-2-methyl)propyl]-aminocrotonate,
4,5-dihydro-2-(1-imidazolylmethyl)thianaphthene-6-yl-methyl (S)-(+)-N-[(1-t-butoxycarbonyl-2-methyl)-propyl]aminocrotonate,
4,5-dihydro-2-(1-imidazolylmethyl)thianaphthene-6-yl-methyl (R)-(-)-N-[(1-t-butoxycarbonyl-2-methyl)propyl]-aminocrotonate,
4,5-dihydro-2-[α-(1-imidazolyl)benzyl]thianaphthene-6-yl-methyl (S)-(+)-N-[(1-t-butoxycarbonyl-2-methyl)-propyl]aminocrotonate,
4,5-dihydro-2-[α-(1-imidazolyl)benzyl]thianaphthene-6-yl-methyl (R)-(-)-N-[(1-t-butoxycarbonyl-2-methyl)-propyl]aminocrotonate,
3-[4-(imidazo-[1,2-a]pyridine-6-yl-methyl)phenyl]-2-propen-1-yl (S)-(+)-N-[(1-t-butoxycarbonyl-2-methyl)-propyl]aminocrotonate, and
3-[4-(imidazo-[1,2-a]pyridine-6-yl-methyl)phenyl]-2-propen-1-yl (R)-(-)-N-[(1-t-butoxycarbonyl-2-methyl)-propyl]aminocrotonate.

It is preferable that the reaction of the acetoacetic ester and amio acid ester for obtaining the optical active β-aminoacrylic acid ester derivative of formula (I-4) be carried out without solvents or in inactive solvents. Examples of the inactive solvents are aromatic hydrocarbons such as benzene and toluene, nitriles such as acetonitrile, halogenated hydrocarbons such as chloroform, and ethers such as tetrahydrofuran. The reaction is usually carried out at 0-100 °C, preferably at 10-70 °C.

The reaction of the β-ketocarboxylic acid ester derivative of formula (I-3) with the optical active β-aminoacrylic acid ester derivative of formula (I-4) for obtaining the optical active 1,4-dihydropyridine derivative of formula (I) can be carried out by reacting both components. In order to carry out the reaction smoothly, it is preferable to add a base to the reaction mixture. Examples of the base include n-butyl lithium, lithium diiso prolylamide, phenyl magnesium halide, metal sodium, and sodium hydride. It is preferable that the amount of the base be 0.5 to 1.5 equivalents to the optical active β-aminoacrylic acid ester derivative of formula (I-4).

Furthermore it is preferable that the above reaction be carried out in non-protonic solvents, for example, ether solvents such as diethyl ether and tetrahydrofuran, and aromatic solvents such as benzene and toluene. The reaction is usually carried out at -120 °C to 150 °C, preferably -90 °C to -20 °C.

In order to increase the yield of the reaction product, it is preferable that the reaction be carried out under an anhydrous condition and under an inert atmosphere by using an inert gas such as a nitrogen gas and an argon gas.

The reaction product obtained by the above reaction is so easily decomposed at room temperature that it is extremely difficult to identify the chemical structure. However, a mass spectrum of the reaction product indicates the chemical structure thereof is as shown below:

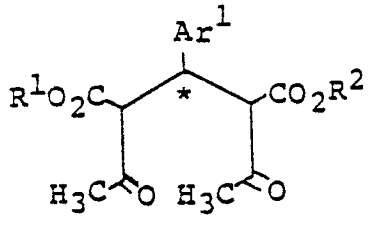

* optically active cite

wherein $Ar^1$, $R^1$ and $R^2$ are respectively the same as defined previously.

The above reaction product is allowed to react with an amine or an acid-added salt thereof, whereby an optical active 1,4-dihydropyridine derivative of formula (II) can be produced.

The amine and the acid-added salt thereof are, for example, ammonia, ammonium acetate, and ammonium chloride, which are industrially available.

In carrying out the reaction, the above mentioned amine can be used as a reaction solvent when used in an excessive amount. Alcohols such as ethanol, methanol and propanol, ethers such as diethyl ether and tetrahydrofuran, and hydrocarbons such as hexane, pentane, toulene and benzene, can also be employed as the reaction solvents.

The reaction is usually carried out at 0° C to 60° C, preferably at room temperature because of the convenience for carrying out the reaction.

The product obtained by the above reaction is isolated and purified by extraction, recrystallization and chromatography in combination.

The optical active 1,4-dihydropyridine derivatives of formula (I) show a hypotensivie action in a test by using male spontaneously hypertensive rats (SHR), and an action of inhibiting KCl-induced contraction in aorta in a test by usig thoracic aortas of rabbits.

Furthermore, the optical active 1,4-dihydropyridine derivatives of formula (I) show high inhibiting activities in a test of platelet aggregation-inhibiting activity in vitro by using rabbits.

Example 1

(+)-3-[4-(1-imidazolylmethyl)phenyl]-2-propen-1-yl methyl-1,4-dihydro-2,6-dimethyl-4-(3-nitrophenyl)-pyridine-3,5-dicarboxylate:

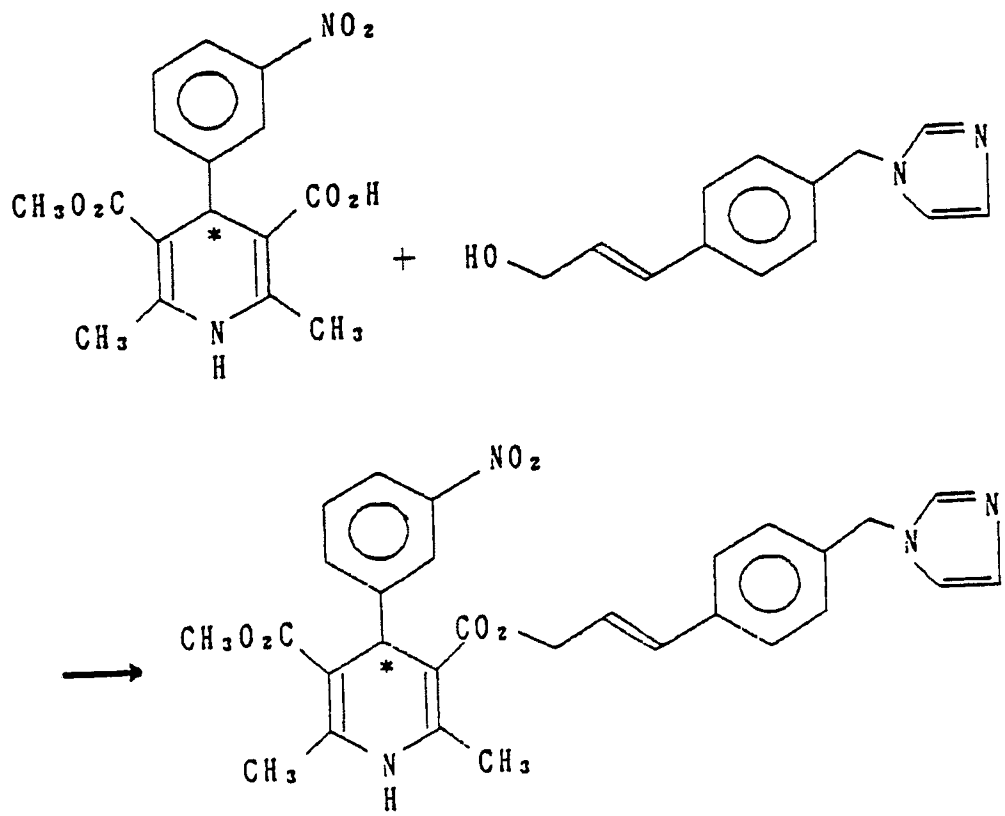

* optically active cite

1.329 g (4 mM) of (-)-1,4-dihydro-2,6-dimethyl-5-methoxycarbonyl-4-(3-nitrophenyl)pyridine-3-carboxylic acid, 1.028 g (4.8 mM) of 3-[4-(1-imidazolylmethyl)phenyl]-2-propen-1-ol, 1.444 g (7 mM) of DCC, and 0.538 g (4.4 mM) of N,N-dimethylaminopyridine were mixed and refluxed in a dried dichloromethane for 5 hours.

The solvent was distilled away, and the residue was purified by chromatography on a silica gel column, whereby the captioned compound was obtained. The yield was 1.898 g (89.8%).
Melting point (°C): 175.0-176.6 (decomposed)

| Mass Spectrometric Analysis : | Molecular formula $C_{29}H_{28}N_4O_6$ | |
|---|---|---|
| | Calcd.<br>Found | 528.20085<br>528.19892 |

$[\alpha]_D^{25}$ = +10.0° [c=0.535, methanol]
IR ($cm^{-1}$, KBr)
νCO 1696
$\nu NO_2$ 1528, 1350
NMR $\delta CDCl_3$ 2.37 (3H, s), 2.39 (3H, s), 3.64 (3H, s), 4.66 (1H, dd, J=14Hz), 4.76 (1H, dd, J=14Hz), 5.13 (2H, s), 5.13 (1H, s), 5.93 (1H, s), 6.23 (1H, dt. J=16Hz, 6Hz), 6.50 (1H, d, J=16Hz), 6.93 (1H, s), 7.13 (2H, d, J=8Hz), 7.13 (1H, s), 7.33 (2H, d, J=8Hz), 7.35 (1H, t, J=8Hz), 7.64 (1Hz, d, J=8Hz), 7.69 (1H, s), 7.97 (1H, d, J=8Hz), 8.13 (1H, s).

Example 2

(-)-3-[4-(-(1-imidazolylmethyl)phenyl]-2-propen-1-yl methyl-1, 4-dihydro-2,6-dimethyl-4-(3-nitrophenyl)-pyridine-3,5-dicarboxylate:

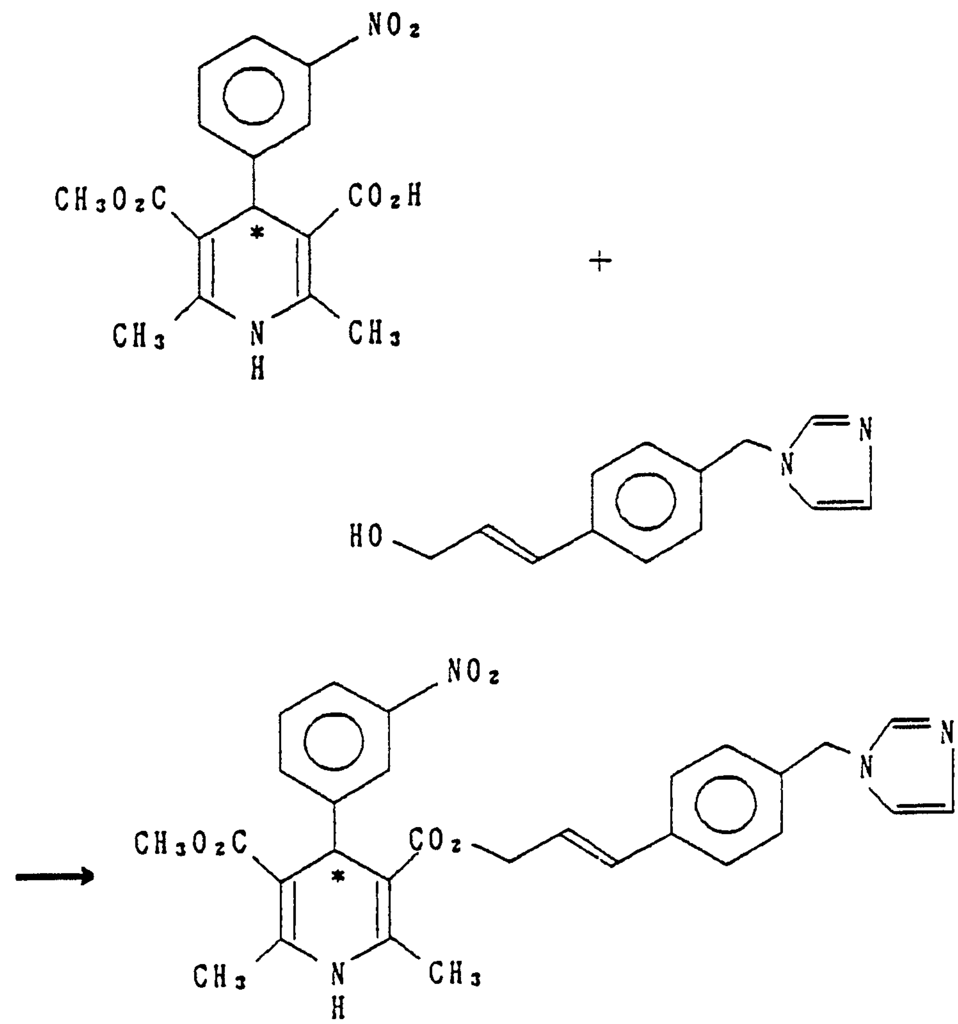

* optically active cite

1.329 g (4 mM) of (+)-1,4-dihydro-2,6-dimethyl-5-methoxycarbonyl-4-(3-nitrophenyl)pyridine-3-carboxylic acid, 1.028 g (4.8 mM) of 3-[4-(1-imidazolylmethyl)phenyl]-2-propen-1-ol, 1.444 g (7 mM) of DCC, and 0.538 g (4.4 mM) of N,N-dimethylaminopyridine were mixed and refluxed in a dried dichloromethane for 5 hours. The solvent was distilled away, and the residue was purified by chromatography on a silica gel column, whereby the captioned compound was obtained. The yield was 1.860 g (88.0%).
Melting point (°C) 175.8-176.3 (decomposed)

| Mass spectrometric Analysis : | Molecular formula $C_{29}H_{28}N_4O_6$ | |
|---|---|---|
| | Calcd. | 528.20085 |
| | Found | 528.19869 |

$[\alpha]_D^{25}$ = -9.8° [c=0.564, methanol]
IR (cm$^{-1}$, KBr)
$\nu$CO 1696
$\nu NO_2$ 1528, 1350
NMR $\delta CDCl_3$ 2.37 (3H, s), 2.39 (3H, s), 3.64 (3H, s), 4.66 (1H, dd, J=14Hz, 7Hz), 4.76 (1H, dd, J=14Hz, 7Hz), 5.13 (2H, s), 5.13 (1H, s), 5.93 (1H, s), 6.23 (1H, dt. J=16Hz, 6 Hz), 6.50 (1H, d, J=16Hz), 6.93 (1H,

s), 7.13 (2H, d, J=8Hz), 7.13 (1H, s), 7.33 (2H, d, J=8Hz), 7.35 (1H, t, J=8Hz), 7.64 (1Hz, d, J=8Hz), 7.69 (1H, s), 7.97 (1H, d, J=8Hz), 8.13 (1H, s).

Example 3

(+)-3-[4-(1-imidazolylmethyl)phenyl]-2-propen-1-yl methyl-1,4-dihydro-2,6-dimethyl-4-(3-nitro-phenyl)-pyridine-3,5-dicarboxylate:

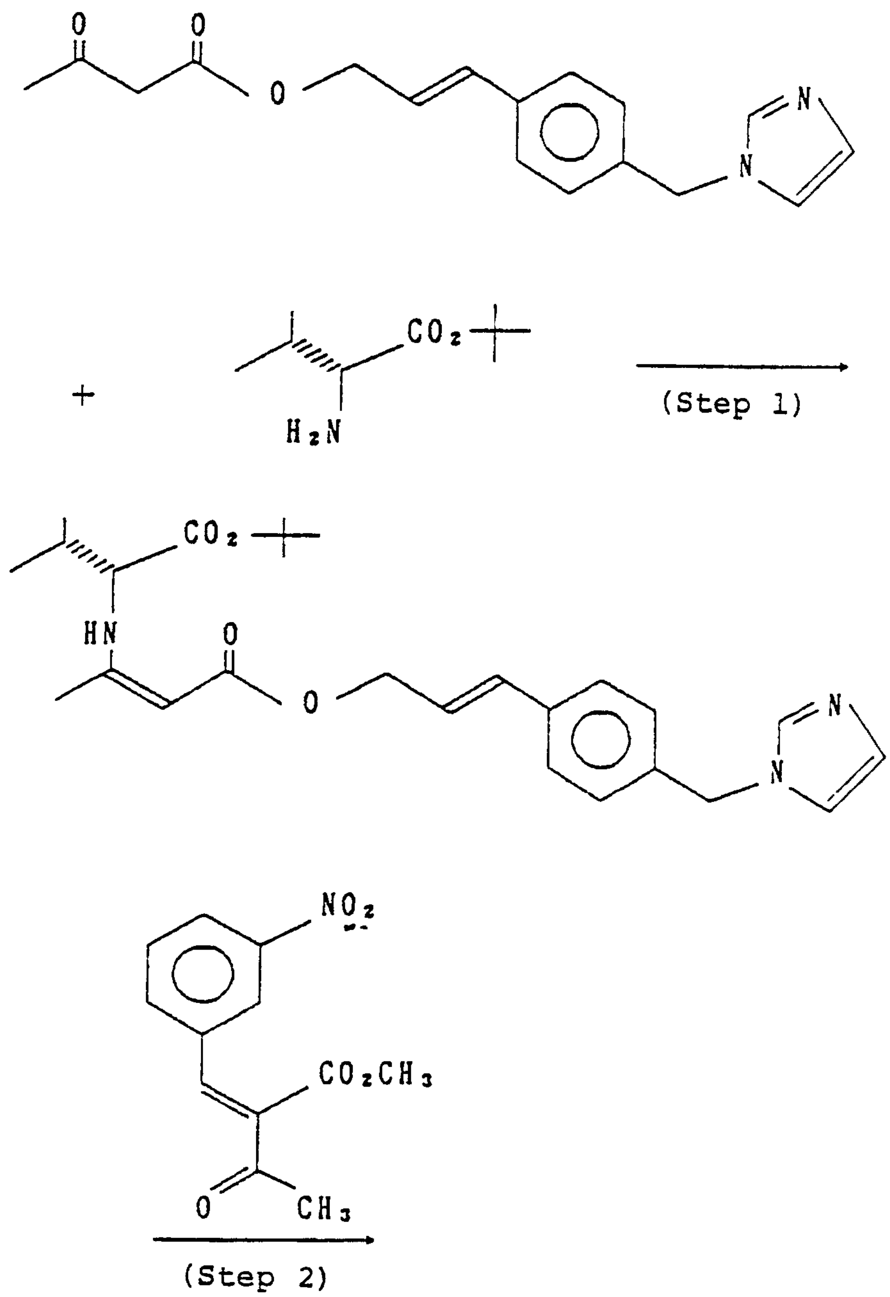


[Step 1]

0.416 g (2.4 mM) of D-valine-t-butyl ester was mixed with 0.597 g (2 mM) of 3-[4-(1-imidazolylmethyl)-phenyl]-2-propen-1-yl acetoacetate. The mixture was stirred for 24 hours. To this mixture, 10 ml of dried

benzene was added.

The mixture was then dried over anhydrous sodium sulfurate. The benzene was then distilled away, whereby 3-[4-(1-imidazolylmethyl)phenyl]-2-propen-1-yl (-)-(R)-N-[(1-t-butoxycarbonyl-2-methyl)propyl]-aminocrotonate was obtained. The yield was 0.898 g (99%).

$[\alpha]_D^{25}$ = -67.4° [c=0.9595, $CHCl_3$]

NMR $\delta CDCl_3$ 1.02 (6H, d, J=7Hz), 1.47 (9H, s), 1.88 (3H, s), 2.11-2.24 (1H, m), 3.79 (1H, dd, J=10Hz, 5Hz), 4.57 (1H, s), 4.73 (2H, d, J=7Hz), 5.11 (2H, s), 6.33 (1H, dt, J=17Hz, 7Hz), 6.61 (1H, d, J=17Hz), 6.90 (1H, s), 7.10 (2H, d, J=9Hz), 7.11 (1H, s), 7.37 (2H, d, J=9Hz), 7.61 (1H, s) 8.87 (1H, d, J=10Hz).

[Step 2]

0.898 g (1.98 mM) of 3-[4-(1-imidazolylmethyl)phenyl]-2-propen-1-yl (-)-(R)-N-[(1-t-butoxycarbonyl-2-methyl)propyl]aminocrotonate was dissolved in 10 ml of dried tetrahydrofuran under an inert atmosphere using an Ar gas, and the solution was cooled to -75° C.

A tetrahydrofuran solution containing 1.2 equivalents of phenyl magnesium bromide, prepared from 77 mg (3.17 mg•atom), 75 mg (0.4 mM) of 1,2-dibromoethane, 374 mg (2.38 mM) of bromobenzene, and 5 mℓ of dried tetrahydrofuran, was added dropwise to the above solution. After the dropwise addition of the terahydrofuran solution, the mixture was stirred for 1 hour. To this mixture, a dried tetrahydrofuran solution containing 449 mg (1.8 mM) of 2-(3-nitrobenzylidene)acetoacetate was added dropwise.

After the above mixture was stirred at -75° C for 20 hours, 5.4 mℓ of 1N HCl was added to the mixture and the temperature of the mixture was raised to room temperature. The mixture consisted of an organic layer and an aqueous layer.

The organic layer was separated from the mixture and the aqueous layer was extracted with tetrahydrofuran, and the tetrahydrofuran containing an extract was added to the organic layer. To this mixture, 5.4 mℓ of 1N HCl was added, and the mixture was stirred at room temperature for 3 hours. The mixture was extracted with chloroform. The chloroform containing an extract was dried over anhydrous sodium sulfate. The chloroform was distilled away from the mixture under reduced pressure, whereby an oily material was obtained.

The thus obtained oily material was dissolved in 10 ml of ethanol, and 700 mg (9 mM) of ammonium acetate was added thereto. The mixture was stirred at room temperature for 1 hour, and the ethanol was distilled away under reduced pressure to obtain a residue. The residue was dissolved in chloroform to form a solution. The solution was washed with a saturated aqueous solution of sodium hydrogencarbonate, and then with water, and dried over anhydrous sodium sulfate. The chloroform was distilled away under reduced pressure. The residue was purified by chromatography on a silica gel column, and recrystallized from methanol, whereby the captioned compound was obtained. The yield was 713 mg (75%). The analysis data of this compound are exactly the same as those of the compound obtained in Example 36.

1. Test for hypotensive activity

The test was carried out by employing spontaneously hypertensive rats (aparalytic SHR; male) according to Nakao et al method.

300 µg/kg of the compound to be tested was administrated into the abdominal aorta of SHR through a canula (previously inserted). The blood pressures in the whole body of the rats were measured with a pressure transducer (MPU-0.5, made by Nihon Koden K.K.). The results are shown in Table 1.

Table 1

| Compounds | MaxΔMBP (mmHg) | Peak (min) | T 1/2 (min) | HR (beats/min) |
|---|---|---|---|---|
| Example 36 | 51.4 | 0.9 | 3.4 | 49.0 |
| Example 37 | 10.8 | 0.6 | 0.4 | 3.7 |

2. Test for activity of inhibiting KCl-induced contraction

Herical strips of thoracic aortas of rabbits (Japanese white rabbit; male) were mounted vertically in organ baths of Krebs-Henselit solution at 37° C for 90 minutes, and then 30 mM of KCl was added to the baths. After 30 minutes, the compound to be tested was added to the baths, and the variations in the tension were recorded by an isometric kymograph, so that the inhibitory activity of the compound was determined in 60 minutes. The results of the test are shown in Table 2.

Table 2

| Compounds | Dosage (M) | N | Inhibitory Effect on KCℓ-induced contraction(%) |
|---|---|---|---|
| Example 36 | $3x10^{-6}$ | 7 | 51.1 ± 11.6 |
| Example 37 | $3x10^{-6}$ | 5 | 16.8 ± 9.8 |

* The dosage is determined in such a manner that the reaction mixture has the above molar concentraion.

3. Test for platelet aggregation-inhibiting activity of rabit

A blood of a rabbit (Japanese white; male; 2.5 - 3.0 kg) was exsanguinated from a carotid of the rabbit, and nine parts of the blood were mixed with one part of a 3.8% aqueous solution of sodium citrate. The mixture was centrifuged at 1100 rpm at 20° C for 15 minutes. The upper layer is a platelet rich plasma (RRP), and the lower layer was centrifuged at 2500 rpm at 20° C for 10 minutes, so that a platelet poor plasma (PPP) was obtained.

10 μℓ of a solution of the compound to be tested with a concentration of $10^{-4}$ mol/ℓ was added to 200 μℓ of PRP, and the mixture was subjected to incubation for 30 minutes. To the mixture was added 10 μℓ of a platelet activating factor (PAF)(10 μg/mℓ), or arachidonic acid (AA)(1 mM at final concentration). The extent of the aggregation was measured by Agricometer (NKK, PAT-4A). The 50% Platelet aggregation-inhibiting concentration ($IC_{50}$ to each aggregation agent of each compound is shown in Table 3.

Table 3

| Compounds | $IC_{50}$ (M) | |
|---|---|---|
| | PAF(10 μg/ml) | AA(1 mM) |
| Example 36 | $1.1 \times 10^{-4}$ | $1.4 \times 10^{-4}$ |
| Example 37 | $3.2 \times 10^{-6}$ | $1.6 \times 10^{-4}$ |

## Claims

1. An optical active 1,4-dihydropyridine derivative having formula (I):

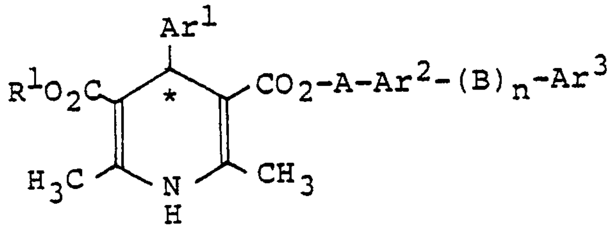

(I)

wherein Ar[1] represents an aromatic hydrocarbon group or an aromatic heteromonocyclic or heterobicyclic group containing therein 1 to 3 atoms selected from the group consisting of oxygen, sulfur and nitrogen; $R^1$ represents a hydrocarbon group which may have one or more substituents; A represents (i) a straight chain or branched chain unsaturated hydrocarbon group, (ii) a cyclic unsaturated hydrocarbon group, or (iii) a group selected from the group consisting of -R-O-N = CH-, -R-N = N-, -R-CH = N- and -R-N = CH-, in which R is an alkylene group having 1 to 6 carbon atoms; B represents an alkylene or alkenylene group having 1 to 3 carbon atoms, which may have a substituent; $Ar^2$ represents an aromatic hydrocarbon group or a heterocyclic group; $Ar^3$ represents a heterocyclic group which may have one or more substituents; n is 0 or 1; and * indicates an optically active cite.

2. The optical active 1,4-dihydropyridine derivatives as claimed in Claim 1, wherein said aromatic hydrocarbon group represented by $Ar^1$ is selected from the group consisting of a phenyl group and a naphthyl group.

3. The optical active 1,4-dihydropyridine derivatives as claimed in Claim 1, wherein said aromatic group represented by $Ar^1$ is a phenyl group.

4. The optical active 1,4-dihydropyridine derivatives as claimed in Claim 1, wherein said aromatic heteromonocyclic or heterobicyclic group represented by $Ar^1$ is selected from the group consisting of a pyridyl group, a benzoxazolyl group, a quinolyl group, a furyl group, a thienyl group, an isoquinolyl, a benzothiazolyl group, a benzoxadiazolyl group and a benzothiadizolyl group.

5. The optical active 1,4-dihydropyridine derivatives as claimed in Claim 1, wherein said aromatic heteromonocyclic or heterobicyclic group represented by $Ar^1$ is selected from the group consisting of a pyridyl group, a benzoxazolyl group, a quinolyl group, a furyl group, and a thienyl group.

6. The optical active 1,4-dihydropyridine derivatives as claimed in Claim 1, wherein said aromatic hydrocarbon group or said aromatic heteromonocyclic or heterobicyclic group represented by $Ar^1$ has a substitutent selected from the group consisting of a nitro group, a trihalomethyl group, a cyano group, a halogen, a phenoxy group, an azido group, an amido group, an alkoxyl group having 1 to 4 carbon atoms, a lower alkyl group having 1 to 4 carbon atoms, an alkylthio group, a lower alkoxy-carbonyl group, and a lower alkylcarbonyl group.

7. The optical active 1,4-dihydropyridine derivatives as claimed in Claim 6, wherein the substituent of said aromatic hydrocarbon group or aromatic heteromonocyclic or heterobicyclic group represented by $Ar^1$ is selected from the group consisting of a nitro group, trihalomethyl group, a cyano group, a halogen, and a phenoxy group.

8. The optical active 1,4-dihydropyridine derivatives as claimed in Claim 1, wherein said aromatic hydrocarbon group or aromatic heteromonocyclic or heterobicyclic group represented by $Ar^1$ is selected from the group consisting of 2-nitrophenyl group, 3-nitrophenyl group, 4-nitrophenyl group, 2-cyanophenyl group, 2,3-dichlorophenyl group, 2-furyl group, 2-thienyl group, 3-thienyl group, 1-naphthyl group, 4-quinolyl group, 3-azidophenyl group, 3-trifluoromethylphenyl group, 4-methoxyphenyl group, 4-methylthiophenyl group, 3-pyridyl group, 4-methylphenyl group and 3-trichloromethylphenyl group.

9. The optical active 1,4-dihydroxypyridine derivatives as claimed in Claim 1, wherein said hydrocarbon group represented by $R^1$ is a straight, branched or cyclic saturated or unsaturated hydrocarbon group having 1 to 6 carbon atoms.

10. The optical active 1,4-dihydroxypyridine derivatives as claimed in Claim 1, wherein said hydrocarbon group represented by $R^1$ is a straight, branched or cyclic saturated or unsaturated hydrocarbon group having 1 to 6 carbon atoms, which may have a substituent selected from the group consisting of an alkoxyl group having 1 to 6 carbon atoms, a thioalkoxyl group, and a mono- or di-substituted amino group.

11. The optical active 1,4-dihydroxypyridine derivatives as claimed in Claim 1, wherein the substituent of said hydrocarbon group represented by $R^1$ is selected from the group consisting of a halogen, a cyano group, a nitro group, a nitrato group, a hydroxyl group, a phenoxy group, a disubstituted amino group, a phenylthio group, a piperidino group, a pyrrolidino group, a substituted piperazinyl group, a morpholino group, and a phenyl group, said phenyl group may further have one or two substituents selected from the

group consisting of a halogen, a cyano group, a trihalomethyl group, an azido group, a disubstituted amino group, an alkyl group having 1 to 4 carbon atoms, an alkoxyl group having 1 to 4 carbon atoms, a lower alkylthio group, a lower alkylcarbonyl group, a lower alkoxycarbonyl group, a carbamoyl group, a halomethyl group, and a hydroxymethyl group.

12. The optical active 1,4-dihydroxypyridine derivatives as claimed in Claim 10, wherein said hydrocarbon group represented by $R^1$ is an alkoxyalkyl group.

13. The optical active 1,4-dihydroxypyridine derivatives as claimed in Claim 12, wherein the substituent of said hydrocarbon group represented by $R^1$ is a disubstituted amino group.

14. The optical active 1,4-dihydroxypyridine derivatives as claimed in Claim 1, wherein said hydrocarbon group represented by $R^1$ is a group represented by $-A-Ar^2-(B)_n-Ar^3$.

15. The optical active 1,4-dihydroxypyridine derivatives as claimed in Claim 9, wherein said hydrocarbon group represented by $R^1$ is selected from the group consisting of a methyl group, an ethyl group, a propyl group, a n-butyl group, a n-pentyl group, a n-hexyl group, an isopropyl group, an isobutyl group, a cyclopentyl group, a cyclohexyl group, a propenyl group, a 2-butenyl group, a 3-butenyl group, a 2-pentynyl group, a 2,4-hexadienyl group, a 2,4-hexadinyl group, a hexa-2-yn-4-ene group and a hexa-2-en-4-yne group.

16. The optical active 1,4-dihydroxypyridine derivatives as claimed in Claim 10, wherein said hydrocarbon group represented by $R^1$ is selected from the group consisting of a methoxyethyl group, an ethoxyethyl group, a methoxypropyl group, an ethoxypropyl group, a methylthioethyl group, an ethylthioethyl group, a methylthiopropyl group, an ethylthiopropyl group, a phenylthioethyl group, a 2-(N-methylamino) ethyl group, a 2-(N,N-dimethylamino)ethyl group, a 2-(N-phenyl-N-methylamino) ethyl group, a 2-(N-benzyl-N-methylamino) ethyl group, a 2-(4-phenylpiperazin-1-yl)ethyl, a 2-{4-(diphenylmethyl)piperazinyl}ethyl, a 2-morpholinoethyl group, an N-benzylpyrrolidin-3-yl, and an N-benzyl-piperidin-3-yl group.

17. The optical active 1,4-dihydroxypyridine derivatives as claimed in Claim 1, wherein said hydrocarbon group represented by A is a straight, branched or cyclic unsaturated hydrocarbon group in which at least an unsaturated bond thereof is conjugated with said aromatic group hydrocarbon or heterocyclic group represented by $Ar^2$.

18. The optical active 1,4-dihydroxypyridine derivatives as claimed in Claim 17, wherein said hydrocarbon group represented by A is a straight or branched unsaturated hydrocarbon group having 3 to 12 carbon atoms, which may have a substituent selected from the group consisting of a halogen, a phenyl group, a pyridyl group, a thienyl group, a furyl group, a cyano group, an alkylcarbonyl group, and an alkoxylcarbonyl group.

19. The optical active 1,4-dihydroxypyridine derivatives as claimed in Claim 17, wherein said hydrocarbon group represented by A is a cyclic unsaturated hydrocarbon group having 4 to 12 carbon atoms, which may have a substituent selected from the group consisting of a halogen, a phenyl group, a pyridyl group, a thienyl group, a furyl group, a cyano group, an alkylcarbonyl group, and alkoxylcarbonyl group.

20. The optical active 1,4-dihydroxypyridine derivatives as claimed in claim 18, wherein said straight or branched unsaturated hydrocarbon group represented by A is selected from the group consisting of:

$-CH_2-CH{=}CH-$ , $-CH_2-(CH{=}CH)_2$ ,

$-CH_2-C{\equiv}C-$ , $-CH_2-(C{\equiv}C)_2$ , $-CH_2-(CH{=}CH)_3$ ,

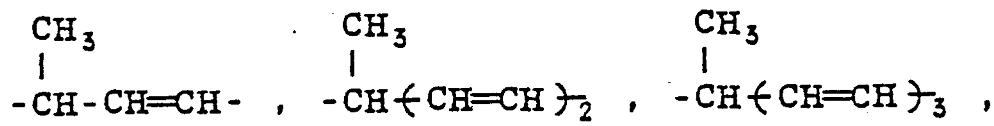

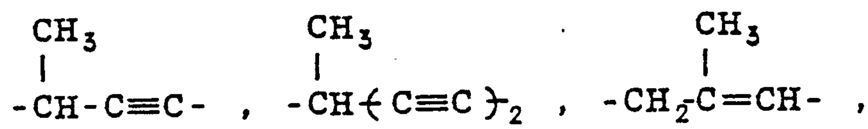

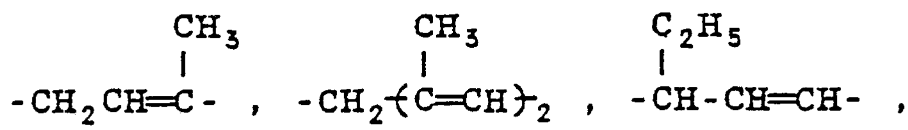

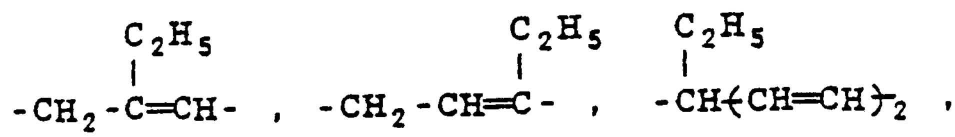

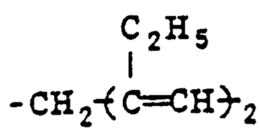 , $-CH_2-CF{=}CH-$ , 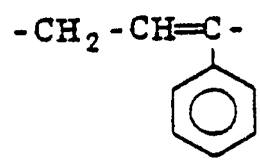 ,

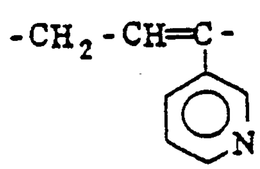 , $-CH_2-CH{=}CH-C{\equiv}C-$ ,

$-CH_2CH_2-CH{=}CH-$ , $-CH_2CH_2-O-N{=}CH-$ ,

$-CH_2CH_2-N{=}CH-$ , $-CH_2CH_2CH{=}N-$ , $-CH_2CH_2-N{=}N-$ and

$-CH_2-C{\equiv}C-CH{=}CH-$ .

21. The optical active 1,4-dihydroxypyridine derivatives as claimed in Claim 1, wherein said aromatic group or heterocyclic group represented by $Ar^2$ is a group selected from the group consisting of o-, m- or p-phenylene group, a furanediyl group, a thiophenediyl group, a pyridinediyl group and a pyrrolediyl group, which may further form a 5- or 6-membered condensed ring in combination with said hydrocarbon group represented by A or a part thereof through a member selected from the group consisting of a methylene group, an ethylene group, a vinyl group, oxygen, sulfur and an amino group.

22. The optical active 1,4-dihydroxypyridine derivatives as claimed in Claim 1, wherein said aromatic group or heterocyclic group represented by $Ar^2$ is selected from the group consisting of:

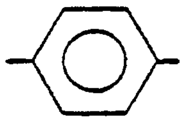, 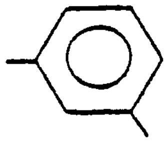, 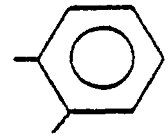, 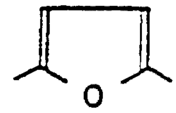,

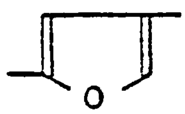, 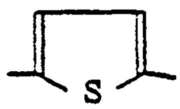, 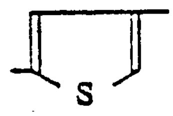, 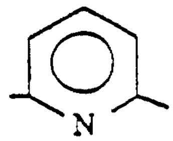,

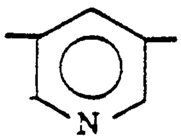, 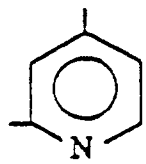, 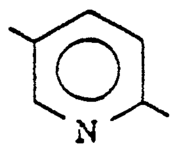, 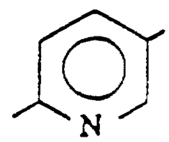,

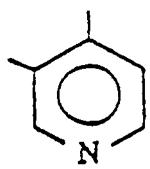, 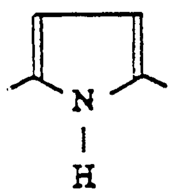, 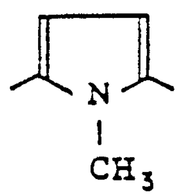,

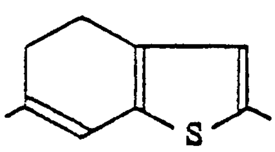, 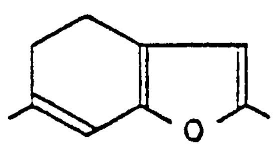, 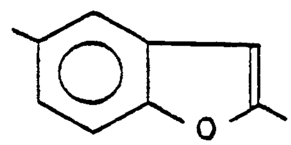,

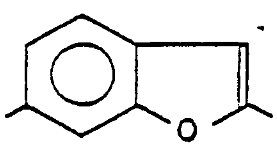, 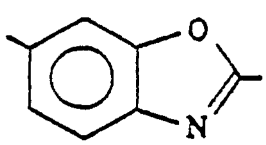, 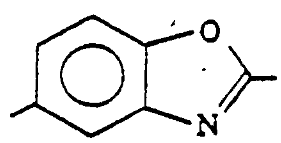,

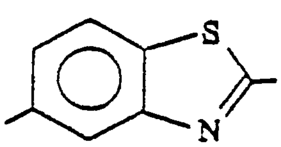 and 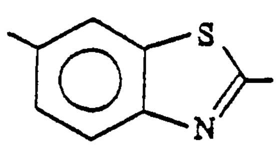 .

23. The optical active 1,4-dihydroxypyridine derivatives as claimed in Claim 1, wherein said hydrocarbon group represented by B has a substituent selected from the group consisting of a lower alkyl group having 1 to 4 carbon atoms, a cyclic hydrocarbon group, an aryl group, and an aralkyl group.

24. The optical active 1,4-dihydroxypyridine derivatives as claimed in Claim 23, wherein said alkylene or alkenylene group is selected from the group consisting of a methylene group, an ethylene group, a

trimethylene group, an ethylidene group, a benzylidene group, a vinylene group, a cylcohexylmethylene group and a 2-phenyl-ethylidene group.

25. The optical active 1,4-dihydroxypyridine derivatives as claimed in Claim 1, wherein said heterocyclic group represented by $Ar^3$ is a 5- to 6-membered heterocyclic ring including at least an atom selected from the group consisting of nitrogen, oxygen and sulfur.

26. The optical active 1,4-dihydroxypyridine derivatives as claimed in Claim 25, wherein said 5- to 6-membered aromatic heterocyclic ring is selected from the group consisting of a 2-pyrrolyl group, a 2-pyridyl group, a 3-pyridyl group, a 4-pyridyl group, an imidazolyl group, a 2-oxazolyl group, a 4-oxazolyl group, a 2-thiazolyl group, a triazolyl group, and a tetrazolyl group.

27. The optical active 1,4-dihydroxypyridine derivatives as claimed in Claim 1, wherein said hydrocarbon group represented by B or a part thereof forms a 5- or 6-membered ring in combination with said aromatic hydrocarbon group or heterocyclic group represented by $Ar^2$ or with said heterocyclic group represented by $Ar^3$ through a member selected from the group consisting of a methylene group, an ethylene group, and a vinylene group.

28. The optical acive 1,4-dihydroxypyridine derivatives as claimed in Claim 27, wherein said 5- or 6-membered ring is selected from the group consisting of:

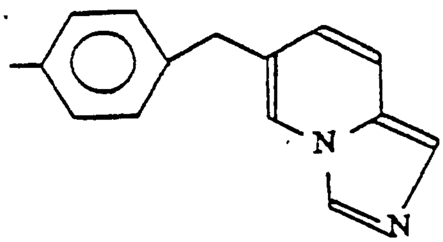

,

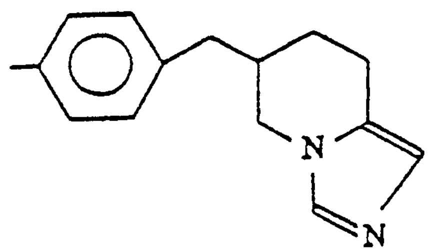

,

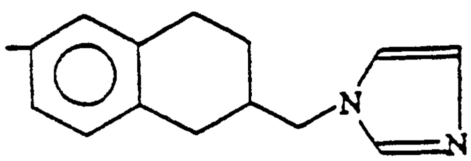

and

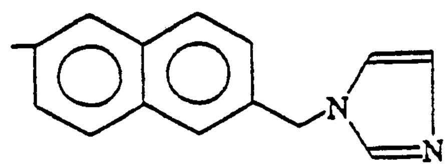

.